# EUROPÄISCHE PATENTSCHRIFT

(11) **EP 1 220 850 B1**
(45) Veröffentlichungstag und Bekanntmachung des Hinweises auf die Patenterteilung: **08.10.2003**
(21) Anmeldenummer: 00966005.1
(22) Anmeldetag: 14.09.2000
(51) Int. Cl.: C07D 321/00, C11B 9/00

(54) **1,4-DIOXACYCLOALKAN-2-ONE UND 1,4-DIOXACYCLOALKEN-2-ONE**
1,4-DIOXACYCLOALKANE-2-ONE AND 1,4-DIOXACYCLOALKENE-2-ONE
1,4-DIOXACYCLOALCAN-2-ONE ET 1,4-DIOXACYCLOALCEN-2-ONE

(30) Priorität: 27.09.1999 DE 19946128
(43) Veröffentlichungstag der Anmeldung: 10.07.2002
(73) Patentinhaber: Symrise GmbH & Co. KG, 37603 Holzminden (DE)
(72) Erfinder: EH, Marcus, 37603 Holzminden (DE); WÖRNER, Peter, 37603 Holzminden (DE)
(74) Vertreter: Eisenführ, Speiser & Partner
(86) Internationale Anmeldenummer: EP0008973
(87) Internationale Veröffentlichungsnummer: WO01023373

(56) Entgegenhaltungen:
- EP-A- 0 884 315
- CHEMICAL ABSTRACTS, vol. 116, no. 28, 1992 Columbus, Ohio, US; abstract no. 59418j, Seite 869; XP002163324 & PL 153 102 A (POLITECHNIKA LODZKA) 29. März 1991 (1991-03-29)
- CHEMICAL ABSTRACTS, vol. 67, no. 23, 1967 Columbus, Ohio, US; abstract no. 53587m, CH. PAQUOT ET AL.: "SYNTHESIS A. PROPERTIES OF THE ISOMERIC 14-HYDROXY-4-OXATETRADECANOIC A. 2-METHYL-13-HYDROXY-3-OXATRIDECANOIC ACIDS." Seite 5019; XP002163325 & REV. FR. CORPS GRAS, Bd. 14, Nr. 3, 1967, Seiten 167-73, FRANCE

## Beschreibung

Die Erfindung betrifft neue 1,4-Dioxacycloalkan-2-one und 1,4-Dioxacycloalken-2-one, ihre Herstellung und Verwendung in der funktionellen Parfümerie und in der Feinparfümerie.

Verbindungen mit Moschusgeruch sind begehrte Komponenten in der Duftstoffindustrie. Sie zeichnen sich sowohl durch ihre Eigenschaft, Parfümkompositionen Ausstrahlung zu verleihen als auch durch ihre Fähigkeit als Fixateur zu wirken, aus. Somit kommen heutzutage Moschus Riechstoffe in vielen Parfümkompositionen zum Einsatz.

Typische Moschus-Riechstoffe zeichnen sich durch einen makrocyclischen Ring mit 13 bis 17 C-Atomen aus, welcher als fünktionelle Gruppe ein Keton oder einen Ester trägt. Darüber hinaus sind auch makrocyclische Moschus Riechstoffe, die zwei funktionelle Gruppe tragen, bekannt, z.B. 1,7-Dioxacycloalkan-8-one (EP A 884,315). Allerdings sind die funktionellen Gruppen dieser Moleküle auf beide Hemispheren der Makrocyclen verteilt. Vorzuziehen sind dagegen makrocyclischen Verbindungen in denen die funktionellen Gruppen in einem Teil des Moleküls konzentriert sind, da somit eine stärkere Bindung am aktiven Zentrum des Rezeptors und als Folge davon ein niedrigerer Schwellenwert erwartet werden kann.

C. Paquot et al. beschreiben in Rev. Fr. Corps Gras 14/3, 168 (1967) die Verbindung 3-Methyl-1,4-dioxacyclotetradecan-2-on, welche einen angenehmen und starken Moschusgeruch aufweist.

In PL 153102 wird ein Herstellverfahren für die Verbindung 1,4-Dioxacycloheptadecan-2-on beschrieben, welche als Komponente von Geruchskompositionen verwendet wird und einen starken, intensiven und reinen Moschusgeruch aufweist.

Aufgrund der zum Teil hohen Kosten der Ausgangsprodukte für die Herstellung und des recht hohen Syntheseaufwandes ist die Anzahl an makrocyclischen Verbindungen, die dem Parfümeur für die Komposition von Parfüms zur Verfügung stehen, relativ beschränkt (K. Bauer, D. Garbe, H. Surburg, Common Fragrance and Flavor Materials, Wiley-VCH, 3^{rd} Edition, 1997, 67 bis 68 und 117 bis 122). Es besteht ein dringender Bedarf an weiteren makrocyclischen Verbindungen, die in einer effizienten Synthese aus günstigen Ausgangsprodukten herstellbar sind und darüber hinaus mit ihren originellen Dufteigenschaften die Möglichkeiten des Parfümeurs erweitern.

Die Klasse der naturähnlichen makrocyclischen Moschusriechstoffe wird in Zukunft immer mehr an Bedeutung gewinnen, da die synthetischen Moschusverbindungen der nitroaromatischen und polycyclischen Reihe persistent und lipophil sind, so daß diese Verbindungen sich in aquatischen Nahrungsketten und Fettgewebe anreichern (H. Brunn, G. Rimkus, Ernährungs-Umschau 1996, 43, 442 bis 449; H. Brunn, G. Rimkus, Emährungs-Umschau 1997, 44, 4 bis 9).

Es bestand daher die Aufgabe, durch neue makrocyclische Moschusverbindungen mit originellen geruchlichen Eigenschaften die für die Komposition von Parfüms zur Verfügung stehende Rohstoff-Palette zu erweitern.

Die neuen Moschus-Riechstoffe sollen außerdem kostengünstig herstellbar sein.

Es wurden neue 1,4-Dioxacycloalkan-2-one und 1,4-Dioxacycloalken-2-one der Formel 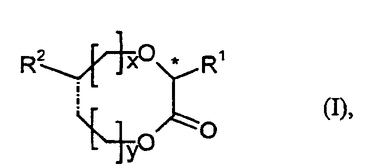 gefunden, worin
die gestrichelte Bindung eine Einfach oder E/Z-Doppelbindung ist,
wobei für den Fall, daß eine Doppelbindung im Ring vorliegt die Verbindungen in der E und Z-Form vorliegen können, und
Verbindungen mit einem chiralen Zentrum sowohl in der (R)- oder (S)-Form als auch als Enantiomerengemisch vorliegen können,
- R¹ und R²: gleich oder verschieden sind und Wasserstoff oder Niederalkyl bedeuten,
- x: für eine gesättigte Alkylenkette mit 1 bis 4 Kohlenstoffatomen und
- y: für eine gesättigte Alkylenkette mit 4 bis 10 Kohlenstoffatomen steht,
ausgewählt aus der Gruppe, die aus
3-Methyl-1,4-dioxa-(E/Z)-6-cyclopentadecen-2-on
3-Methyl-1,4-dioxacyclopentadecan-2-on
1,4-Dioxa-(E/Z)-6-cyclopentadecen-2-on
1,4-Dioxacyclopentadecan-2-on
3-Methyl-1,4-dioxa-(E/Z)-6-cyclohexadecen-2-on
3-Methyl-1,4-dioxacyclohexadecan-2-on
1,4-Dioxa-(E/Z)-6-cyclohexadecen-2-on
1,4-Dioxacyclohexadecan-2-on
1,4-Dioxa-(E/Z)-7-cyclohexadecen-2-on
3-Methyl-1,4-dioxa-(E/Z)-7-cyclohexadecen-2-on
besteht.

Die erfindungsgemäßen 1,4-Dioxacycloalkan-2-one und 1,4-Dioxacycloalken-2-one, in der die funktionellen Gruppen in enger Nachbarschaft zueinander stehen, erfüllen die gestellte Aufgabe in idealer Weise. Neben parfümistisch interessanten moschusartigen Geruchsnoten zeichnen sie sich durch eine sehr gute Haftfestigkeit verbunden mit einem niedrigen Schwellenwert aus.

Besonders attraktive olfaktorische Geruchseigenschaften haben die gesättigten, an 3-Position methylsubstituierten 1,4-Dioxacycloalkan-2-one, insbesondere 3-Methyl-1,4-dioxacyclopentadecan-2-on und 3-Methyl-1,4-dioxacyclohexadecan-2-on. Sie zeichnen sich durch eine süße, holzig-ambrierte, animalische, erogene und damit sehr naturhafte Moschusnote aus. Das olfaktorische Profil der ungesättigten 3-Methyl-1,4-dioxacyloalken-2-one ist sehr ähnlich dem der gesättigten Verbindungen, allerdings ist die Intensität geringer. Zum Vergleich wurden die analogen 1,4-Dioxacycloalkan-2-one und 1,4-Dioxacycloalken-2-one ohne Methylsubstitution an 3-Position synthetisiert. Überraschenderweise traten bei den ungesättigten 1,4-Dioxacycloalken-2-onen die erogenen und animalischen Aspekte in den Hintergrund zugunsten von metallisch, bügeleisenhaften Geruchsbeschreibungen. Die gesättigten 1,4-Dioxacycloalkan-2-one hingegen und hierbei insbesondere 1,4-Dioxacyclohexadecan-2-on zeichnen sich wieder durch eine sehr schöne natürliche Moschushaftigkeit aus.

Im Vergleich zu den bekannten 1,4-Dioxacycloheptadecan-2-on und 3-Methyl-1,4-dioxacyclotetradecan-2-on haben die erfindungsgemäßen 1,4-Dioxacycloalkan-2-one und 1,4-Dioxacycloalken-2-one überraschenderweise eine sehr viel naturhaftere Moschusnote, gepaart mit Nitromoschus und Ambrett Moschus Aspekten.

Es wurde ein Verfahren zur Herstellung der erfindungsgemäßen, 1,4-Dioxacycloalkan-2-one und 1,4-Dioxacycloalken-2-one gefunden,
das dadurch gekennzeichnet ist, daß in 2-Position derivatisierbare Alkylcarbonsäuren oder deren Ester der Formel 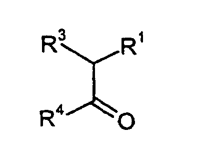 worin
- R¹: die oben genannte Bedeutung hat, und
- R³: für OH, Cl, Br und
- R⁴: für OH, OMe oder OEt steht,
eingesetzt werden, die im 1. Schritt verethert, in einem 2. Schritt verestert und in einem 3. Schritt der Ring durch Olefimmetathese zu den ungesättigten 1,4-Dioxacycloalken-2-onen geschlossen wird, die dann gegebenenfalls in einem 4. Schritt zu den gesättigten 1,4-Dioxacycloalkan-2-onen hydriert werden.

Im 1. Schritt wird für den Fall R³ = OH und R⁴ = OMe oder OEt mit einem Äquivalent Natriumhydrid als Base in Tetrahydrofuran als Lösungsmittel deprotoniert. Anschließend erfolgt die Zugabe von 1,5-Äquivalenten des ,ω-Alkenhalogenids wonach die Reaktionsmischung unter Rückfluß erhitzt wird, so daß die 2-Alkenyloxycarbonsäureester erhalten werden (Tetrahedron Lett. 1976, 17, 3535).

Im Falle der α-Halogencarbonsäuren (R³ = Cl, Br und R⁴ = OH) als Ausgangsverbindungen benötigt man 2 bis 3 Äquivalente Natriumhydrid, wobei zuerst das α,ω-Alkenol deprotoniert wird, bevor man die α-Halogencarbonsäure zugibt. Auch diese Mischung wird unter Rückfluß erhitzt, um die 2-Alkenyloxycarbonsäuren zu bekommen (J. Org. Chem. 1998, 63, 3160).

Die auf oben beschriebene Weise synthetisierten 2-Alkenyloxycarbonsäuren (R⁴ = OH) werden im 2. Schritt unter Zugabe von 1 bis 3 Äquivalenten des entsprechenden α,ω-Alkenols und 0,1 bis 5 Mol% p-Toluolsulfonsäure arn Wasserabscheider mit Toluol als Schlepper zu den doppelt terminal ungesättigten 2-Alkenyloxycarbonsäurealkenylestern verestert.

Liegen die 2-Alkenyloxycarbonsäureester (R⁴ = OMe, OEt) vor, so werden diese zuerst mit 1,5 bis 2 Äquivalenten LiOH in Methanol/Wasser (Verhältnis:3:1) verseift, um anschließend mit 1 bis 3 Äquivalenten des entsprechenden α,ω-Alkenols und 0,1 bis 5 Mol% 0p-Toluolsulfonsäure am Wasserabscheider zu den doppelt terminal ungesättigten 2-Alkenyloxycarbonsäurealkenylestem verestert zu werden.

Als Zwischenprodukte entstehen hier die neuen doppelt terminal ungesättigten 2-Alkenyloxycarbonsäurealkenylester der Formel 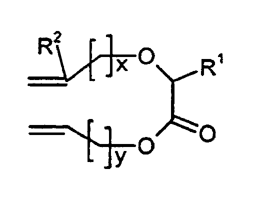 worin R¹, R², x und y die oben genannte Bedeutung haben.

Die Herstellung der erfindungsgemäßen 1,4-Dioxacycloalken-2-one (3. Schritt) erfolgt ausgehend von den doppelt terminal ungesättigten 2-Alkenyloxycarbonsäurealkenylestern in einer Ringschluß Olefinmetathese (US 4,490,404; D. Tetrahedron Lett., 1980, 21, 1715; JP 10 175,882). Hierzu wird der 2-Alkenyloxycarbonsäurealkenylester in einer 0,01 bis 0,003 molaren Dichlormethanlösung mit 0,1 bis 0,5 Äquivalenten Titantetraisopropoxid für eine Stunde am Rückfluß erhitzt. Anschließende Zugabe von 0,5 bis 5 Mol% Benzyliden-bis-(tricyclohexylphosphin)-dichlororuthenium (Grubbs-Katalysator) und erneutes auf Rückflußtemperatur erhitzen für 8 bis 48 Stunden (Synthesis, 1997, 792; Synlett, 1997, 1010) liefert die erfindungsgemäßen 1,4-Dioxacycloalken-2-one.

Die Hydrierung (4. Schritt) bei Normal-Wasserstoffdruck und Raumtemperatur mit 1 bis 5 Gewichtsprozent Pd/C in Isopropanol liefert ausgehend von den erfindungsgemäßen 1,4-Dioxacycloalken-2-onen die erfindungsgemäßen 1,4-Dioxacycloalkan-2-one.

Das erfindungsgemäße Verfahren kann am Beispiel des 3-Methyl-1,4-dioxacyclopentadecan-2-ons durch das folgende Formelschema erläutert werden:

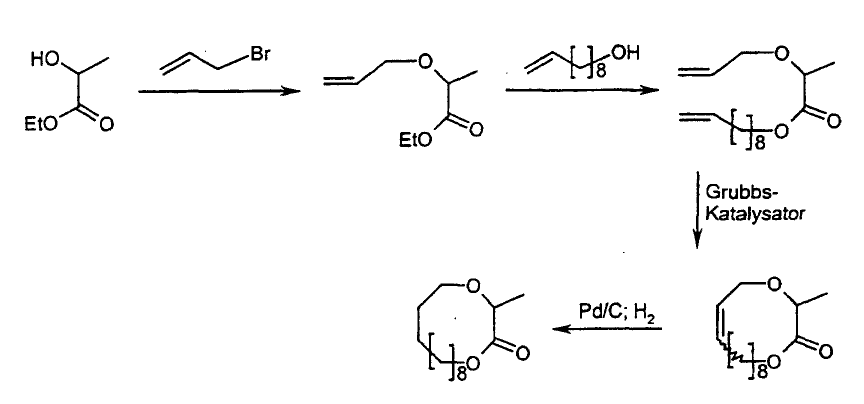

Es wurde ein weiteres Verfahren zur Herstellung der erfindungsgemäßen chiralen 3-Alkyl-1,4-dioxacycloalkan-2-one und 3-Alkyl-1,4-dioxacycloalken-2-one der Formel 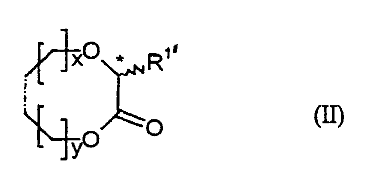 gefunden, worin
die gestrichelte Bindung eine Einfach oder E/Z-Doppelbindung ist,
wobei für den Fall, daß eine Doppelbindung im Ring vorliegt die Verbindungen in der E und Z-Form vorliegen können,
und die Verbindungen mit einem chiralen Zentrum in der (R)- oder (S)-Form vorliegen,
R^{1'} die in Anspruch für den Rest R¹ eines jeweiligen makrocyclischen 1,4-Dioxacycloalkan-2-ons oder 1,4-Dioxacycloalken-2-ons genannte Bedeutung hat, und
x die in Anspruch 1 für das jeweilige makrocyclische 1,4-Dioxacycloalkan-2-on oder 1,4-Dioxacycloalken-2-on genannte Bedeutung hat
y die in Anspruch 1 für das jeweilige makrocyclische 1,4-Dioxacycloalkan-2-on oder 1,4-Dioxacycloalken-2-on genannte Bedeutung hat
das dadurch gekennzeichnet ist, daß als Edukte (S)-2- oder (R)-2-Hydroxycarbonsäurealkylester der Formel 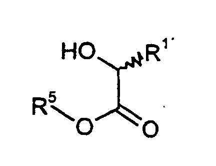 worin
- R¹: die oben genannte Bedeutung hat, und
- R⁵: für ein C₁ bis C₈ Alkylrest steht,
eingesetzt werden, die in einem 1. Schritt unter sauren, nicht racemisierenden Bedingungen verethert, in einem 2. Schritt unter Lewissäure-Katalyse umgeestert und in einem 3. Schritt der Ring durch Olefinmetathese zu den ungesättigten 3-Alkyl-1,4dioxacycloalken-2-onen geschlossen wird, die dann gegebenenfalls in einem 4. Schritt zu den gesättigten 3-Alkyl-1,4-dioxacycloalkan-2-onen hydriert werden.

Im 1. Schritt wird die O-Alkylierung der (R)- oder (S)-2-Hydroxycarbonsäurealkylester über die Trichloracetimidat-Methode (Tetrahedron Lett., 1988, 29, 4139-4142) durchgeführt. Hierzu werden die chiralen 2-Hydroxycarbonsäurealkylester in Cyclohexan mit 2 bis 3 Äquivalenten des Alkenyltrichloracetimidats und 5 bis 15 Mol% Trifluormethansulfonsäure versetzt. Nach 16 bis 24 Stunden bei Raumtemperatur erhält man die chiralen (R)- oder (S)-2-Alkenyloxycarbonsäurealkylester. Die Enantiomerenüberschüsse dieser Verbindungen liegen bei ≥ 95%.

In der nachfolgenden Umesterung (2. Schritt) wird der chirale 2-Alkenyloxycarbonsäureester mit 1 bis 3 Äquivalenten des entsprechenden ,ω-Alkenols und unter Zugabe von 1 bis 10 Mol% Titantetraisopropoxid zur Reaktion gebracht (Tetrahedron Lett., 1998, 4223-4226). Somit erhält man die chiralen, doppelt terminal ungesättigten 2-Alkenyloxycarbonsäurealkenylester.

Die chiralen doppelt terminal ungesättigten 2-Alkenyloxycarbonsäurealkenylester der Formel 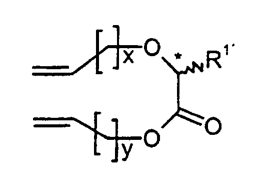 worin R^{1'}, x und y die oben genannte Bedeutung haben,
sind neu.

Die Enantiomerenüberschüsse dieser Verbindungen liegen bei > 95%.

Die Herstellung der erfindungsgemäßen chiralen 3-Alkyl-1,4-dioxacycloalken-2-one und 3-Alkyl-1,4-dioxacycloalkan-2-one erfolgt nach den oben beschriebenen Methoden, über Ringschluß Olefinmetathese (3. Schritt) und anschließender Hydrierung (4. Schritt).

Die Enantiomerenüberschüsse der gesättigten 3-Alkyl-1,4-dioxacycloalkan-2-one liegen bei ≥ 95%.

Das erfindungsgemäße Verfahren kann am Beispiel des (S)-(-)-3-Methyl-1,4-dioxacyclopentadecan-2-ons durch das folgende Formelschema erläutert werden:

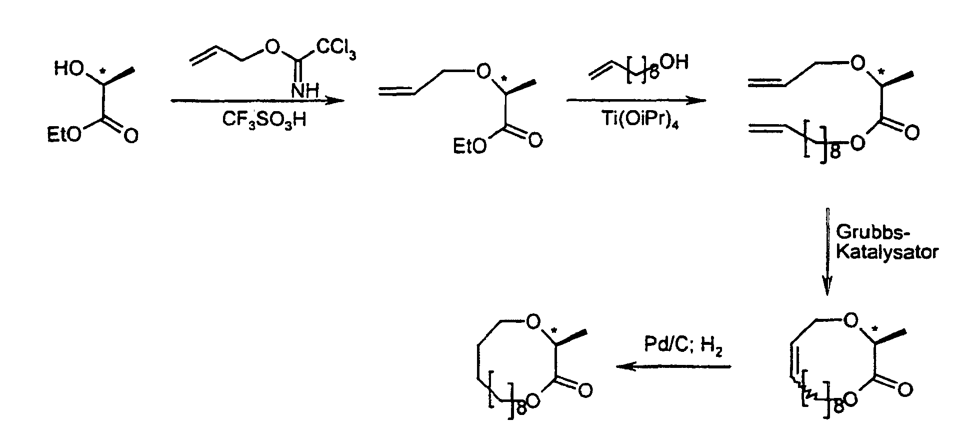

Im Einzelnen seien die folgenden chiralen 3-Alkyl-1,4-dioxacycloalkan-2-one und 3-Alkyl-1,4-dioxacycloalken-2-one genannt:
(S)-(-)-3-Methyl-1,4-dioxa-(E/Z)-6-cyclopentadecen-2-on
(S)-(-)-3-Methyl-1,4-dioxacyclopentadecan-2-on
(S)-(-)-3-Methyl-1,4-dioxa-(E/Z)-6-cyclohexadecen-2-on
(S)-(-)-3-Methyl-1,4-dioxacyclohexadecan-2-on
(R)-(+)-3-Methyl-1,4-dioxa-(E/Z)-6-cyclopentadecen-2-on
(R)-(+)-3-Methyl-1,4-dioxacyclopentadecan-2-on
(R)-(+)-3-Methyl-1,4-dioxa-(E/Z)-6-cyclohexadecen-2-on
(R)-(+)-3-Methyl-1,4-dioxacyclohexadecan-2-on

Die erfindungsgemäßen 1,4-Dioxacycloalkan-2-one und 1,4-Dioxacycloalken-2-one können dabei als Einzelstoffe in einer Vielzahl von Produkten verwendet werden; besonders vorteilhaft lassen sie sich mit anderen Riechstoffen zu neuartigen Parfümkompositionen kombinieren.

Durch die Verwendung der erfindungsgemäßen 1,4-Dioxacycloalkan-2-one und 1,4-Dioxacycloalken-2-one lassen sich in der Regel bereits in geringer Dosierung in den resultierenden Parfümkompositionen feine, erogene Moschusnoten erzielen, wobei der geruchliche Gesamteindruck auffallend harmonisiert, die Austrahlung wahrnehmbar erhöht und die Fixierung, d.h. das Haftvermögen des Parfümöles, deutlich verstärkt wird.

Beispiele für Riechstoffe, mit denen die erfindungsgemäßen 1,4-Dioxacycloalkan-2-one und 1,4-Dioxacycloalken-2-one vorteilhaft kombiniert werden können, finden sich z.B. in S. Arctander, Perfume and Flavor Materials, Vol. I und II, Montclair, N. J., 1969, Selbstverlag oder K. Bauer, D. Garbe und H. Surburg, Common Fragrance and Flavor Materials, 3^{rd}. Ed., Wiley-VCH, Weinheim 1997.

Im einzelnen seien genannt:
Extrakte aus natürlichen Rohstoffen wie Etherische Öle, Concretes, Absolues, Resine, Resinoide, Balsame, Tinkturen wie z. B. Ambratinktur; Amyrisöl; Angelicasamenöl; Angelicawurzelöl; Anisöl; Baldrianöl; Basilikumöl; Baummoos -Absolue; Bayöl; Beifußöl; Benzoeresin; Bergamotteöl; Bienenwachs-Absolue; Birkenteeröl; Bittermandelöl; Bohnenkrautöl; Buccoblätteröl; Cabreuvaöl; Cadeöl; Calmusöl; Campheröl; Canangaöl; Cardamomenöl; Cascarillaöl; Cassiaöl; Cassie-Absolue; Castoreum-absolue; Cedemblätteröl; Cedernholzöl; Cistusöl; Citronellöl; Citronenöl; Copaivabalsam; Copaivabalsamöl; Corianderöl; Costuswurzelöl; Cuminöl; Cypressenöl; Davanaöl; Dillkrautöl; Dillsamenöl; Eau de brouts-Absolue; Eichenmoos-Absolue; Elemiöl; Estragonöl; Eucalyptus-citriodora-Öl; Eucalyptusöl; Fenchelöl; Fichtennadelöl; Galbanumöl; Galbanumresin; Geraniumöl; Grapefruitöl; Guajakholzöl; Gurjunbalsam; Gurjunbalsamöl; Helichrysum-Absolue; Helichrysumöl; Ingweröl; Iriswurzel-Absolue; Iriswurzelöl; Jasmin-Absolue; Kalmusöl; Kamillenöl blau; Kamillenöl römisch; Karottensamenöl; Kaskarillaöl; Kiefernadelöl; Krauseminzöl; Kümmelöl; Labdanumöl; Labdanum-Absolue; Labdanumresin; Lavandin-Absolue; Lavandinöl; Lavendel-Absolue; Lavendelöl; Lemongrasöl; Liebstocköl; Limetteöl destilliert; Limetteöl gepreßt; Linaloeöl; Litsea-cubeba-Öl; Lorbeerblätteröl; Macisöl; Majoranöl; Mandarinenöl; Massoirindenöl; Mimosa-Absolue; Moschuskörneröl; Moschustinktur; Muskateller-Salbei-Öl; Muskatnußöl; Myrrhen-Absolue; Myrrhenöl; Myrtenöl; Nelkenblätteröl; Nelkenblütenöl; Neroliöl; Olibanum-Absolue; Olibanumöl; Opopanaxöl; Orangenblüten-Absolue; Orangenöl; Origanumöl; Palmarosaöl; Patchouliöl; Perillaöl; Perubalsamöl; Petersilienblätteröl; Petersiliensamenöl; Petitgrainöl; Pfefferminzöl; Pfefferöl; Pimentöl; Pineöl; Poleyöl; Rosen-Absolue; Rosenholzöl; Rosenöl; Rosmarinöl; Salbeiöl dalmatinisch; Salbeiöl spanisch; Sandelholzöl; Selleriesamenöl; Spiklavendelöl; Sternanisöl; Styraxöl; Tagetesöl; Tannennadelöl; Tea-tree-Öl; Terpentinöl; Thymianöl; Tolubalsam; Tonka-Absolue; Tuberosen-Absolue; Vanilleextrakt; Veilchenblätter-Absolue; Verbenaöl; Vetiveröl; Wacholderbeeröl; Weinhefenöl; Wermutöl; Wintergrünöl; Ylangöl; Ysopöl; Zibet-Absolue; Zimtblätteröl; Zimtrindenöl; sowie Fraktionen davon, bzw. daraus isolierten Inhaltsstoffen;
Einzel-Riechstoffe aus der Gruppe der Kohlenwasserstoffe, wie z.B. 3-Caren; α-Pinen; β-Pinen; α-Terpinen; γ-Terpinen; p-Cymol; Bisabolen; Camphen; Caryophyllen; Cedren; Farnesen; Limonen; Longifolen; Myrcen; Ocimen; Valencen; (E,Z)-1,3,5-Undecatrien;
der aliphatischen Alkohole wie z. B. Hexanol; Octanol; 3-Octanol; 2,6-Dimethylheptanol; 2-Methylheptanol, 2-Methyloctanol; (E)-2-Hexenol; (E)- und (Z)-3-Hexenol; 1-Octen-3-ol; Gemisch von 3,4,5,6,6-Pentamethyl-3/4-hepten-2-ol und 3,5,6,6-Tetramethyl-4-methyleneheptan-2-ol; (E,Z)-2,6-Nonadienol; 3,7-Dimethyl-7-methoxyoctan-2-ol; 9-Decenol; 10-Undecenol; 4-Methyl-3-decen-5-ol; der aliphatischen Aldehyde und deren 1,4-Dioxacycloalken-2-one wie z. B. Hexanal; Heptanal; Octanal; Nonanal; Decanal; Undecanal; Dodecanal; Tridecanal; 2-Methyloctanal; 2-Methylnonanal; (E)-2-Hexenal; (Z)-4-Heptenal; 2,6-Dimethyl-5-heptenal; 10-Undecenal; (E)-4-Decenal; 2-Dodecenal; 2,6,10-Trimethyl-5,9-undecadienal; Heptanaldiethylacetal; 1,1-Dimethoxy-2,2,5-trimethyl-4-hexen; Citronellyloxyacetaldehyd;
der aliphatischen Ketone und deren Oxime wie z.B. 2-Heptanon; 2-Octanon; 3-Octanon; 2-Nonanon; 5-Methyl-3-heptanon; 5-Methyl-3-heptanonoxim; 2,4,4,7-Tetramethyl-6-octen-3-on; der aliphatischen schwefelhaltigen Verbindungen wie z.B. 3-Methylthiohexanol; 3-Methylthiohexylacetat; 3-Mercaptohexanol; 3-Mercaptohexylacetat; 3-Mercaptohexylbutyrat; 3-Acetylthiohexylacetat; 1-Menthen-8-thiol;
der aliphatischen Nitrile wie z.B. 2-Nonensäurenitril; 2-Tridecensäurenitril; 2,12-Tridecensäurenitril; 3,7-Dimethyl-2,6-octadiensäurenitril; 3,7-Dimethyl-6-octensäurenitril;
der aliphatischen Carbonsäuren und deren Ester wie z.B. (E)- und (Z)-3-Hexenylformiat; Ethylacetoacetat; Isoamylacetat; Hexylacetat; 3,5,5-Trimethylhexylacetat; 3-Methyl-2-butenylacetat; (E)-2-Hexenylacetat; (E)- und (Z)-3-Hexenylacetat; Octylacetat; 3-Octylacetat; 1-Octen-3-ylacetat; Ethylbutyrat; Butylbutyrat, ; Isoamylbutyrat; Hexylbutyrat; (E)- und (Z)-3-Hexenylisobutyrat; Hexylcrotonat; Ethylisovalerianat; Ethyl-2-methylpentanoat; Ethylhexanoat; Allylhexanoat; Ethylheptanoat; Allylheptanoat; Ethyloctanoat; Ethyl-(E,Z)-2,4-decadienoat; Methyl-2-octinat; Methyl-2-noninat; Allyl-2-isoamyloxyacetat; Methyl-3,7-dimethyl-2,6-octadienoat;
der acyclischen Terpenalkohole wie z.B. Citronellol; Geraniol; Nerol; Linalool; Lavadulol; Nerolidol; Famesol; Tetrahydrolinalool; Tetrahydrogeraniol; 2,6-Dimethyl-7-octen-2-ol; 2,6-Dimethyloctan-2-ol; 2-Methyl-6-methylen-7-octen-2-ol; 2,6-Dimethyl-5,7-octadien-2-ol; 2,6-Dimethyl-3,5-octadien-2-ol; 3,7-Dimethyl-4,6-octadien-3-ol; 3,7-Dimethyl-1,5,7-octatrien-3-ol 2,6-Dimethyl-2,5,7-octatrien-1-ol; sowie deren Formiate, Acetate, Propionate, Isobutyrate, Butyrate, Isovalerianate, Pentanoate, Hexanoate, Crotonate, Tiglinate, 3-Methyl-2-butenoate;
der acyclischen Terpenaldehyde und -ketone wie z.B. Geranial; Neral; Citronellal; 7-Hydroxy-3,7-dimethyloctanal; 7-Methoxy-3,7-dimethyloctanal; 2,6,10-Trimethyl-9-undecenal; Geranylaceton; sowie die Dimethyl- und Diethylacetale von Geranial, Neral, 7-Hydroxy-3,7-dimethyloctanal;
der cyclischen Terpenalkohole wie z.B. Menthol; Isopulegol; alpha-Terpineol; Terpinenol-4; Menthan-8-ol; Menthan-1-ol; Menthan-7-ol; Bomeol; Isoborneol; Linalooloxid; Nopol; Cedrol; Ambrinol; Vetiverol; Guajol; sowie deren Formiate, Acetate, Propionate, Isobutyrate, Butyrate, Isovalerianate, Pentanoate, Hexanoate, Crotonate, Tiglinate, 3-Methyl-2-butenoate;
der cyclischen Terpenaldehyde und -ketone wie z.B. Menthon; Isomenthon; 8-Mercaptomenthan-3-on; Carvon; Campher; Fenchon; alpha-Ionon; beta-Ionon; alpha-n-Methylionon; beta-n-Methylionon; alpha-Isomethylionon; beta-Isomethylionon; alpha-Iron; alpha-Damascon; beta-Damascon; beta-Damascenon; delta-Damascon; gamma-Damascon; 1-(2,4,4-Trimethyl-2-cyclohexen-1-yl)-2-buten-1-on; 1,3,4,6,7,8a-Hexahydro-1,1,5,5-tetramethyl-2H-2,4a-methanonaphthalen-8(5H)-on; Nootkaton; Dihydronootkaton; alpha-Sinensal; beta-Sinensal; Acetyliertes Cedernholzöl (Methylcedrylketon);
der cyclischen Alkohole wie z.B. 4-tert.-Butylcyclohexanol; 3,3,5-Trimethylcyclohexanol; 3-Isocamphylcyclohexanol; 2,6,9-Trimethyl-Z2,Z5,E9-cyclododecatrien-1-ol; 2-Isobutyl-4-methylterrahydro-2H-pyran-4-ol;
der cycloaliphatischen Alkohole wie z.B. alpha,3,3-Trimethylcyclohexylmethanol; 2-Methyl-4-(2,2,3-trimethyl-3-cyclopent-1-yl)butanol; 2-Methyl-4-(2,2,3-trimethyl-3-cyclopent-1-yl)-2-buten-1-ol; 2-Ethyl-4-(2,2,3-trimethyl-3-cyclopent-1-yl)-2-buten-1-ol; 3-Methyl-5-(2,2,3-trimethyl-3-cyclopent-1-yl)-pentan-2-ol; 3-Methyl-5-(2,2,3-trimethyl-3-cyclopent-1-yl)-4-penten-2-ol; 3,3-Dimethyl-5-(2,2,3-trimethyl-3-cyclopent-1-yl)-4-penten-2-ol; 1-(2,2,6-Trimethylcyclohexyl)pentan-3-ol; 1-(2,2,6-Trimethylcyclohexyl)hexan-3-ol;
der cyclischen und cycloaliphatischen Ether wie z.B. Cineol; Cedrylmethylether; Cyclododecylmethylether; (Ethoxymethoxy)cyclododecan; alpha-Cedrenepoxid; 3a,6,6,9a-Tetramethyldodecahydronaphtho[2,1-b]furan; 3a-Ethyl-6,6,9a-trimethyldodecahydronaphtho[2,1-b]furan; 1,5,9-Trimethyl-13-oxabicyclo[10.1.0]trideca-4,8-dien; Rosenoxid; 2-(2,4-Dimethyl-3-cyclohexen-1-yl)-5-methyl-5-(1-methylpropyl)-1,3-dioxan;
der cyclischen Ketone wie z.B. 4-tert.-Butylcyclohexanon; 2,2,5-Trimethyl-5-pentylcyclopentanon; 2-Heptylcyclopentanon; 2-Pentylcyclopentanon; 2-Hydroxy-3-methyl-2-cyclopenten-1-on; 3-Methyl-cis-2-penten-1-yl-2-cyclopenten-1-on; 3-Methyl-2-pentyl-2-cyclopenten-1-on; 3-Methyl-4-cyclopentadecenon; 3-Methyl-5-cyclopentadecenon; 3-Methylcyclopentadecanon; 4-(1-Ethoxyvinyl)-3,3,5,5-tetramethylcyclohexanon; 4-tert.-Pentylcyclohexanon; 5-Cyclohexadecen-1-on; 6,7-Dihydro-1,1,2,3,3-pentamethyl-4(5H)-indanon; 5-Cyclohexadecen-1-on; 8-Cyclohexadecen-1-on; 9-Cycloheptadecen-1-on; Cyclopentadecanon;
der cycloaliphatischen Aldehyde wie z.B. 2,4-Dimethyl-3-cyclohexencarbaldehyd; 2-Methyl-4-(2,2,6-trimethyl-cyclohexen-1-yl)-2-butenal; 4-(4-Hydroxy-4-methylpentyl)-3-cyclohexencarbaldehyd; 4-(4-Methyl-3-penten-1-yl)-3-cyclohexencarbaldehyd;
der cycloaliphatischen Ketone wie z. B. 1-(3,3-Dimethylcyclohexyl)-4-penten-1-on; 1-(5,5-Dimethyl-1-cyclohexen-1-yl)-4-penten-1-on; 2,3,8,8-Tetramethyl-1,2,3,4,5,6,7,8-octahydro-2-naphtalenylmethylketon; Methyl-2,6,10-trimethyl-2,5,9-cyclododecatrienylketon; tert.-Butyl-(2,4-dimethyl-3-cyclohexen-1-yl)keton;
der Ester cyclischer Alkohole wie z.B. 2-tert-Butylcyclohexylacetat; 4-tert Butylcyclohexylacetat; 2-tert-Pentylcyclohexylacetat; 4-tert-Pentylcyclohexylacetat; Decahydro-2-naphthylacetat; 3-Pentyltetrahydro-2H-pyran-4-ylacetat; Decahydro-2,5,5,8a-tetramethyl-2-naphthylacetat; 4,7-Methano-3a,4,5,6,7,7a-hexahydro-5, bzw. 6-indenylacetat; 4,7-Methano-3a,4,5,6,7,7a-hexahydro-5, bzw. 6-indenylpropionat; 4,7-Methano-3a,4,5,6,7,7a-hexahydro-5, bzw. 6-indenylisobutyrat; 4,7-Methanooctahydro-5, bzw. 6-indenylacetat;
der Ester cycloaliphatischer Carbonsäuren wie z. B. Allyl-3-cyclohexylpropionat; Allylcyclohexyloxyacetat; Methyldihydrojasmonat; Methyljasmonat; Methyl-2-hexyl-3-oxocyclopentancarboxylat; Ethyl-2-ethyl-6,6-dimethyl-2-cyclohexencarboxylat; Ethyl-2,3,6,6-tetramethyl-2-cyclohexencarboxylat; Ethyl-2-methyl-1,3-dioxolan-2-acetat;
der aromatischen Kohlenwasserstoffe wie z. B. Styrol und Diphenylmethan;
der araliphatischen Alkohole wie z.B. Benzylalkohol; 1-Phenylethylalkohol; 2-Phenylethylalkohol; 3-Phenylpropanol; 2-Phenylpropanol; 2-Phenoxyethanol; 2,2-Dimethyl-3-phenylpropanol; 2,2-Dimethyl-3-(3-methylphenyl)propanol; 1,1-Dimethyl-2-phenylethylalkohol; 1,1-Dimethyl-3-phenylpropanol; 1-Ethyl-1-methyl-3-phenylpropanol; 2-Methyl-5-phenylpentanol; 3-Methyl-5-phenylpentanol; 3-Phenyl-2-propen-1-ol; 4-Methoxybenzylalkohol; 1-(4-Isopropylphenyl)ethanol;
der Ester von araliphatischen Alkoholen und aliphatischen Carbonsäuren wie z.B.; Benzylacetat; Benzylpropionat; Benzylisobutyrat; Benzylisovalerianat; 2-Phenylethylacetat; 2-Phenylethylpropionat; 2-Phenylethylisobutyrat; 2-Phenylethylisovalerianat; 1-Phenylethylacetat; alpha-Trichlormethylbenzylacetat; alpha,alpha-Dimethylphenylethylacetat; alpha,alpha-Dimethylphenylethylbutyrat; Cinnamylacetat; 2-Phenoxyethylisobutyrat; 4-Methoxybenzylacetat; der araliphatischen Ether wie z.B. 2-Phenylethylmethylether; 2-Phenylethylisoamylether; 2-Phenylethyl-1-ethoxyethylether; Phenylacetaldehyddimethylacetal; Phenylacetaldehyddiethylacetal; Hydratropaaldehyddimethylacetal; Phenylacetaldehydglycerinacetal; 2,4,6-Trimethyl-4-phenyl-1,3-dioxane; 4,4a,5,9b-Tetrahydroindeno[1,2-d]-m-dioxin; 4,4a,5,9b-Tetrahydro-2,4-dimethylindeno[1,2-d]-m-dioxin;
der aromatischen und araliphatischen Aldehyde wie z. B. Benzaldehyd; Phenylacetaldehyd; 3-Phenylpropanal; Hydratropaaldehyd; 4-Methylbenzaldehyd; 4-Methylphenylacetaldehyd; 3-(4-Ethylphenyl)-2,2-dimethylpropanal; 2-Methyl-3-(4-isopropylphenyl)propanal; 2-Methyl-3-(4-tert.-butylphenyl)propanal; 3-(4-tert.-Butylphenyl)propanal; Zimtaldehyd; alpha-Butylzimtaldehyd; alpha-Amylzimtaldehyd; alpha-Hexylzimtaldehyd; 3-Methyl-5-phenylpentanal; 4-Methoxybenzaldehyd; 4-Hydroxy-3-methoxybenzaldehyd; 4-Hydroxy-3-ethoxybenzaldehyd; 3,4-Methylendioxybenzaldehyd; 3,4-Dimethoxybenzaldehyd; 2-Methyl-3-(4-methoxyphenyl)propanal; 2-Methyl-3-(4-methylendioxyphenyl)propanal;
der aromatischen und araliphatischen Ketone wie z.B. Acetophenon; 4-Methylacetophenon; 4-Methoxyacetophenon; 4-tert.-Butyl-2,6-dimethylacetophenon; 4-Phenyl-2-butanon; 4-(4-Hydroxyphenyl)-2-butanon; 1-(2-Naphthalenyl)ethanon; Benzophenon; 1,1,2,3,3,6-Hexamethyl-5-indanylmethylketon; 6-tert.-Butyl-1,1-dimethyl-4-indanylmethylketon; 1-[2,3-dihydro-1,1,2,6-tetramethyl-3-(1-methylethyl)-1H-5-indenyl]ethanon; 5',6',7',8'-Tetrahydro-3',5',5',6',8',8'-hexamethyl-2-acetonaphthon;
der aromatischen und araliphatischen Carbonsäuren und deren Ester wie z.B. Benzoesäure; Phenylessigsäure; Methylbenzoat; Ethylbenzoat; Hexylbenzoat; Benzylbenzoat; Methylphenylacetat; Ethylphenylacetat; Geranylphenylacetat; Phenylethylphenylacetat; Methylcinnmat; Ethylcinnamat; Benzylcinnamat; Phenylethylcinnamat; Cinnamylcinnamat; Allylphenoxyacetat; Methylsalicylat; Isoamylsalicylat; Hexylsalicylat; Cyclohexylsalicylat; Cis-3-Hexenylsalicylat; Benzylsalicylat; Phenylethylsalicylat; Methyl-2,4-dihydroxy-3,6-dimethylbenzoat; Ethyl-3-phenylglycidat; Ethyl-3-methyl-3-phenylglycidat;
der stickstoffhaltigen aromatischen Verbindungen wie z.B. 2,4,6-Trinitro-1,3-dimethyl-5-tert.-butylbenzol; 3,5-Dinitro-2,6-dimethyl-4-tert.-butylacetophenon; Zimtsäurenitril; 5-Phenyl-3-methyl-2-pentensäurenitril; 5-Phenyl-3-methylpentansäurenitril; Methylanthranilat; Methy-N-methylanthranilat; Schiff sche Basen von Methylanthranilat mit 7-Hydroxy-3,7-dimethyloctanal, 2-Methyl-3-(4-tert.-butylphenyl)propanal oder 2,4-Dimethyl-3-cyclohexencarbaldehyd; 6-Isopropylchinolin; 6-Isobutylchinolin; 6-sec.-Butylchinolin; Indol; Skatol; 2-Methoxy-3-isopropylpyrazin; 2-Isobutyl-3-methoxypyrazin;
der Phenole, Phenylether und Phenylester wie z.B. Estragol; Anethol; Eugenol; Eugenylmethylether; Isoeugenol; Isoeugenylmethylether; Thymol; Carvacrol; Diphenylether; beta-Naphthylmethylether; beta-Naphthylethylether; beta-Naphthylisobutylether; 1,4-Dimethoxybenzol; Eugenylacetat; 2-Methoxy-4-methylphenol; 2-Ethoxy-5-(1-propenyl)phenol; p-Kresylphenylacetat;
der heterocyclischen Verbindungen wie z.B. 2,5-Dimethyl-4-hydroxy-2H-furan-3-on; 2-Ethyl-4-hydroxy-5-methyl-2H-furan-3-on; 3-Hydroxy-2-methyl-4H-pyran-4-on; 2-Ethyl-3-hydroxy-4H-pyran-4-on;
der Lactone wie z.B. 1,4-Octanolid; 3-Methyl-1,4-octanolid; 1,4-Nonanolid; 1,4-Decanolid; 8-Decen-1,4-olid; 1,4-Undecanolid; 1,4-Dodecanolid; 1,5-Decanolid; 1,5-Dodecanolid; 1,15-Pentadecanolid; cis- und trans-11-Pentadecen-1,15-olid; cisund trans-12-Pentadecen-1,15-olid; 1,16-Hexadecanolid; 9-Hexadecen-1,16-olid; 10-Oxa-1,16-hexadecanolid; 11-Oxa-1,16-hexadecanolid; 12-Oxa-1,16-hexadecanolid; Ethylen-1,12-dodecandioat; Ethylen-1,13-tridecandioat; Cumarin; 2,3-Dihydrocumarin; Octahydrocumarin.

Die die erfindungsgemäßen 1,4-Dioxacycloalkan-2-one und 1,4-Dioxacycloalken-2-one enthaltenden Parfümöle können in flüssiger Form, unverdünnt oder mit einem Lösungmittel verdünnt für Parfümierungen eingesetzt werden. Geeignete Lösungsmittel hierfür sind z.B. Ethanol, Isopropanol, Diethylenglycolmonoethylether, Glycerin, Propylenglycol, 1,2-Butylenglycol, Dipropylenglycol, Diethylphthalat, Triethylcitrat, Isopropylmyristat usw.

Des weiteren können die die erfindungsgemäßen 1,4-Dioxacycloalkan-2-one und 1,4-Dioxacycloalken-2-one enthaltenden Parfümöle an einem Trägerstoff adsorbiert sein, der sowohl für eine feine Verteilung der Riechstoffe im Produkt als auch für eine kontrollierte Freisetzung bei der Anwendung sorgt. Derartige Träger können poröse anorganische Materialien wie Leichtsulfat, Kieselgele, Zeolithe, Gipse, Tone, Tongranulate, Gasbeton usw. oder organische Materialien wie Hölzer und Cellulosebasierende Stoffe sein.

Die die erfindungsgemäßen 1,4-Dioxacycloalkan-2-one und 1,4-Dioxacycloalken-2-one enthaltenden Parfümöle können auch mikroverkapselt, sprühgetrocknet, als Einschluß-Komplexe oder als Extrusions-Produkte vorliegen und in dieser Form dem zu parfümierenden Produkt hinzugefügt werden.

Gegebenenfalls können die Eigenschaften der derart modifizierten Parfümöle durch sog "Coaten" mit geeigneten Materialien im Hinblick auf eine gezieltere Duftfreisetzung weiter optimiert werden, wozu vorzugsweise wachsartige Kunststoffe wie z.B. Polyvinylalkohol verwendet werden.

Die Mikroverkapselung der Parfümöle kann beispielsweise durch das sogenannte Koazervationsverfahren mit Hilfe von Kapselmaterialien z.B. aus polyurethan-artigen Stoffen oder Weichgelatine, erfolgen. Die sprühgetrockneten Parfümöle können beispielsweise durch Sprühtrocknung einer das Parfümöl enthaltenden Emulsion, bzw. Dispersion hergestellt werden, wobei als Trägerstoffe modifizierte Stärken, Proteine, Dextrin und pflanzliche Gummen verwendet werden können. Einschluß-Komplexe können z.B. durch Eintragen von Dispersionen von dem Parfümöl und Cyclodextrinen oder Harnstoffderivaten in ein geeignetes Lösungsmittel, z.B. Wasser, hergestellt werden. Extrusions-Produkte können durch Verschmelzen der Parfümöle mit einem geeigneten wachsartigen Stoff und durch Extrusion mit nachfolgender Erstarrung, ggf. in einem geeigneten Lösungsmittel, z.B. Isopropanol, erfolgen.

In Parfümkompositionen beträgt die eingesetzte Menge der erfindungsgemäßen 1,4-Dioxacycloalkan-2-one und 1,4-Dioxacycloalken-2-one 0,05 bis 50 Gew.-%, vorzugsweise 0,5 bis 20 %, bezogen auf das gesamte Parfümöl.

Die die erfindungsgemäßen 1,4-Dioxacycloalkan-2-one und 1,4-Dioxacycloalken-2-one enthaltenden Parfümöle können in konzentrierter Form, in Lösungen oder in oben beschriebener modifizierter Form verwendet werden für die Herstellung von z.B. Parfüm-Extraits, Eau de Parfüms, Eau de Toilettes, Rasierwässer, Eau de Colognes, Pre-shave-Produkte, Splash-Colognes und parfümierten Erfrischungstüchern sowie die Parfümierung von sauren, alkalischen und neutralen Reinigungsmitteln, wie z.B. Fußbodenreinigem, Fensterglasreinigem, Geschirrspülmittel, Badund Sanitärreinigem, Scheuermilch, festen und flüssigen WC-Reinigern, pulver- und schaumförmigen Teppichreinigern, flüssigen Waschmitteln, pulverförmigen Waschmitteln, Wäschevorbehandlungsmitteln wie Bleichmittel, Einweichmittel und Fleckenentfemem, Wäscheweichspülern, Waschseifen, Waschtabletten, Desinfektionsmitteln, Oberflächendesinfektionsmitteln sowie von Luftverbesserern in flüssiger, gelartiger oder auf einem festen Träger aufgebrachter Form, Aerosolsprays, Wachsen und Polituren wie Möbelpolituren, Fußbodenwachsen, Schuhcremes sowie Körperpflegemitteln wie z.B. festen und flüssigen Seifen, Duschgelen, Shampoos, Rasierseifen, Rasierschäumen, Badeölen, kosmetischen Emulsionen vom Öl-in-Wasser-, vom Wasser-in-Öl- und vom Wasser-in-Öl-in-Wasser-Typ wie z.B. Hautcremes- und -lotionen, Gesichtscremes und -lotionen, Sonnenschutzcremes-undlotionen, After-sun-cremes und -lotionen, Handcremes und -lotionen, Fußcremes und -lotionen, Enthaarungscremes und-lotionen, After-shave-Cremes und -lotionen, Bräunungscremes und -lotionen. Haarpflegeprodukten wie z.B. Haarsprays, Haargelen, festigen Haarlotionen, Haarspülungen, permanenten und semipermanenten Haarfärbemitteln, Haarverformungsmitteln wie kaltwellen und Haarglättungsmitteln, Haarwässern, Haarcremes und -lotionen, Deodorantien und Antiperspirantien wie z.B. Achselsprays, Roll-ons, Deosticks, Deocremes, Produkten der dekorativen Kosmetik wie z.B. Lidschatten, Nagellacke, Make-ups, Lippenstifte, Mascara sowie von Kerzen, Lampenölen, Räucherstäbchen, Insektiziden, Repellentien, Treibstoffen.

Ein Schwerpunkt der Verwendung der erfindungsgemäßen 1,4-Dioxacycloalkan-2-one und 1,4-Dioxacycloalken-2-one liegt wegen ihrer Stabilität im alkalischen Bereich bei der Parfümierung von Seifen und Waschmitteln. Bei der Verwendung in Waschmittelparfümierungen zeichnen sich die erfindungsgemäßen 1,4-Dioxacycloalkan-2-one und 1,4-Dioxacycloalken-2-one durch eine im Vergleich zu bisher verwendeten Riechstoffen erhöhte Substantivität, d.h. durch ein verstärktes Aufziehvermögen und eine erhöhte Haftung des Riechstoffs auf der gewaschenen Faser, aus.

Die folgenden Verbindungen erläutern die Erfindung:

### Beispiel 1:

### 1,4-Dioxacyclotetradecan-2-on (nitch beansprucht)

2-Bromessigsäurehex-5-enylester: Zu einer auf 0°C abgekühlten Lösung aus 4,1 g (30 mmol) Bromessigsäure, 250 mg (3 mmol) DMAP und 3,3 g (33 mmol) 5-Hexen-1-ol in 30 ml CH₂Cl₂ gibt man 6,2 g (30 mmol) DCC. Nach beendeter Zugabe läßt man auf Raumtemperatur kommen und rührt über Nacht. Ist die Reaktion beendet, filtriert man das ausgefallene Harnstoffderivat ab, engt das Filtrat ein und nimmt den Rückstand in n-Pentan auf. Jetzt wird erneut abfiltriert und das Filtrat wird abschließend noch zweimal mit 0,5N HCl und einmal mit gesättigter NaHCO₃-Lösung gewaschen. Das so erhaltene Rohprodukt wird ohne weitere Reinigung in die nächste Reaktion eingesetzt.

2-(5-Hexenyloxy)-essigsäurehex-5-enylester (x, y = 4, R¹, R²= H): Zu einer Suspension aus 0,95 g (28 mmol) NaH in 20 ml THF werden 3,5 g (34 mmol) 5-Hexen-1-ol, gelöst in 25 ml THF, getropft. Nach beendeter Zugabe gibt man tropfenweise 4,8 g (23 mmol) 2-Bromessigsäurehex-5-enylester, gelöst in 15 ml THF, zu. Anschließend erhitzt man über Nacht am Rückfluß. Nach beendeter Reaktion läßt man abkühlen und quencht mit 2N HCl. Nach erfolgter Phasentrennung wird die wässrige Phase noch einmal mit Et₂O und zweimal mit EtOAc extrahiert. Die vereinigten organischen Phasen werden über Na₂SO₄ getrocknet, abfiltriert und einrotiert. Das Rohprodukt wird an Kieselgel (Cyclohexan/EtOAc = 40:1; R_{f} = 0,26) gereinigt, so daß man als Produkt ein farbloses Öl in einer Ausbeute von 4,8 g (67% über 2 Stufen) erhält.

¹H-NMR (200 MHz, CDCl₃): δ (ppm) = 1.38-1.56 (m, 4H), 1.56-1.76 (m, 4H), 2.0-2.16 (m, 4H), 3.51 (t, J = 6.4 Hz, 2H), 4.07 (s, 2H), 4.17 (t, J = 6.4 Hz, 2H), 4.99-5.08 (m, 4H), 5.73 (ddd, J = 17.2, 10.2, 5.8 Hz, 1H), 5.86 (ddd, J = 17.2, 10.2, 5.8 Hz, 1H).

¹³C-NMR (50 MHz, CDCl₃): δ (ppm) = 25.0, 25.2, 27.9, 28.9, 33.2, 33.4, 64.6, 68.2, 71.6, 114.5, 114.8, 138.1, 138.4, 170.5

Die Ringschluß Olefinmetathese und nachfolgende Hydrierung wurden analog der unter Beispiel 2.1 beschriebenen Vorschriften durchgeführt. Somit sind an dieser Stelle nur die spektroskopischen Daten aufgeführt:
1,4-Dioxa-(E/Z)-9-cyclotetradecen-2-on (x, y = 4, R¹, R² = H):
   Geruch: holzig, cedrig, Patchouly, Moschus.
      Angabe der Isomerie: Überschuß-:Unterschußisomer
      ¹H-NMR (200 MHz, CDCl₃): δ (ppm) = 1.33-1.83 (m, 8H), 1.95-2.16 (m, 4H), 3.53 (t, J = 6.5 Hz, 2H), 4.07:4.05 (s, 2H), 4.17-4.33 (m, 2H), 5.25-5.70 (m, 2H).
      ¹³C-NMR (50 MHz, CDCl₃): δ (ppm) = 25.2, 25.4, 25.6, 26.4, 26.7, 27.9, 63.8:64.6, 69.6: 68.9, 70.7, 71.7, 129.6:130.7, 130.0:131.3, 171.2.
1,4-Dioxacyclotetradecan-2-on (x, y = 4, R¹, R² = H):
   Geruch: holzig, Patchouly, Moschus, erogen, metallisch, an heißes Bügeleisen erinnernd.
      ¹H-NMR (200 MHz, CDCl₃): δ (ppm) = 1.27-1.56 (m, 12H), 1.60-1.77 (m, 2H), 3.53 (t, J = 6.4 Hz, 2H), 4.09 (s, 2H), 4.26 (dd, J = 5.42, 4.42 Hz, 2H).
      ¹³C-NMR (50 MHz, CDCl₃): δ (ppm) =23.9, 24.0, 24.6, 25.3, 25.5, 25.6, 26.9, 27.5, 64.8, 69.4, 70.4, 170.9.

### Beispiel 2.1:

### (RS)-3-Methyl-1,4-dioxacyclopentadecan-2-on

(RS)-2-Allyloxy-propionsäureethylester (x = 1, Y = OEt, R¹ = Me, R² = H): Zu einer Suspension aus 8,0 g (0,2 mol) NaH in 150 ml THF werden 23,6 g (0,2 mol) (S)-(-)-Milchsäureethylester, gelöst in 100 ml THF, gegeben. Nach beendeter Zugabe tropft man 36,3 g (0,3 mol) Allylbromid, gelöst in 10 ml THF, zu. Anschließend gibt man noch etwas KJ hinzu und erhitzt 16 h am Rückfluß. Hiernach läßt man abkühlen und quencht die Reaktion mit 100 ml 2N HCl. Die wässrige Phase wird noch zweimal mit Et₂O extrahiert, bevor die vereinigten organischen Phasen über Na₂SO₄ getrocknet werden. Anschließend wird filtriert und am Rotationsverdampfer zur Trockne eingeengt. Man erhält 31,7 g Rohprodukt (GC-Reinheit 89,0%), welches ohne weitere Reinigung in die nächsten Reaktionen eingesetzt wird.

(RS)-2-Allyloxy-propionsäuredec-9-enylester (x = 1, y = 8, R¹ = Me, R² = H): Man legt 3,5 g rohen (RS)-2-Allyloxy-propionsäureethylester in 24 ml MeOH/H₂O = 3:1 vor, kühlt auf 0°C ab und gibt anschließend 2,2 g (30 mmol) LiOH•H₂O portionsweise zu. Man läßt 5 Minuten weiterrühren bevor man die Eiskühlung entfernt und noch 1 h bei Raumtemperatur rührt. Jetzt wird Methanol am Rotationsverdampfer entfernt und der Rückstand in Wasser aufgenommen. Zunächst wird die wässrige Phase einmal mit Et₂O extrahiert, um dann auf pH = 1 mit 6N HCl angesäuert zu werden. Nachfolgend sättigt man die wässrige Phase mit NaCl und extrahiert dreimal mit EtOAc. Die vereinigten organischen Phasen werden über Na₂SO₄ getrocknet, abfiltriert und am Rotationsverdampfer von Lösungsmittel befreit. Die so erhaltene Carbonsäure wird in 40 ml Toluol gelöst und mit 4,7 g (30 mmol) 9-Decen-1-ol und 0,38 g (2 mmol) p-Toluolsulfonsäure versetzt. Anschließend erhitzt man so lange am Wasserabscheider bis sich keine sichtbaren Mengen Wasser mehr abscheiden. Nach Abkühlen auf Raumtemperatur wird die Reaktionslösung noch einmal mit gesättigter NaHCO₃-Lösung gewaschen, dann über Na₂SO₄ getrocknet, abfiltriert und am Rotationsverdampfer von Lösungsmittel befreit. Nach Flashchromatographie (Cyclohexan/EtOAc = 20:1, R_{f}= 0,31) an Kieselgel erhält man 2,8 g (52,2%) (RS)-2-Allyloxy-propionsäuredec-9-enylester als farbloses Öl.

¹H-NMR (200 MHz, CDCl₃): δ (ppm) = 1.25-1.40 (m, 10H), 1.42 (d, J = 6.9 Hz, 3H), 1.57-1.74 (m, 2H), 1.97-2.13 (m, 2H), 3.94 (ddd, J = 12.5, 5.9, 1.7 Hz, 2H), 4.02 (q, J = 6.7 Hz, 1H), 4.14 (m, 2H), 4.93 (ddd, 1H, J = 10.2, 2.2, 1.1 Hz, 1H), 4.99 (ddd, J = 17.2, 2.2, 1.4 Hz, 1H), 5.20 (ddd, J = 10.2, 1.7, 1.3 Hz, 1H), 5.29 (dq, J = 17.2, 1.7 Hz, 1H), 5.81 (ddd, J = 17.2, 10.2, 6.7 Hz, 1H), 5.93 (dddd, J = 17.2, 10.2, 6.0, 5.2 Hz, 1H).
¹³C-NMR (50 MHz, CDCl₃): δ (ppm) = 18.7, 25.8, 28.5, 28.8, 29.0, 29.1, 29.3, 33.7, 64.9, 71.0, 74.0, 114.1, 117.7, 134.1, 139.1, 173.4.

(RS)-3-Methyl-1,4-dioxa-(E/Z)-6-cyclopentadecen-2-on (x = 1, y = 8, R¹ = Me, R² = H): Zu einer Lösung aus 187 mg (0,7 mmol) (RS)-2-Allyloxy-propionsäuredec-9-enylester in 220 ml CH₂Cl₂ werden 59,6 mg (0,21 mmol) Ti(OiPr)₄ gegeben und 1 h am Rückfluß erhitzt. Anschließend werden 16,4 mg (0,02 mmol) Benzyliden-bis-(tricyclohexylphosphin)-dichlororuthenium, gelöst in 5 ml CH₂Cl₂, zugegeben und 20 h am Rückfluß erhitzt. Nach dem Abkühlen auf Raumtemperatur wird die Reaktionslösung zweimal mit je 50 ml 1N HCl gewaschen, danach trocknet man über Na₂SO₄ bevor über eine kurze Kieselgel-Säule filtriert wird. Jetzt entfernt man das Lösungsmittel am Rotationsverdampfer und erhält 160 mg (95%) eines farblosen Öls.

Geruch: Moschus, süß-blumig, ambriert, erogen, an Ambrett Moschus erinnernd.
Angabe der Isomerie: Überschuß-:Unterschußisomer
¹H-NMR (400 MHz, CDCl₃): δ (ppm) = 1.23-1.46 (m, 10H), 1.40:1,41 (d, J = 6.9:6.9 Hz, 3H), 1.57-1.74 (m, 2H), 1.91-2.14 (m, 2H), 3.86-4.12 (m, 2H), 4.01:4.03 (q, J = 6.9:6.9 Hz, 1H), 4.20-4.44 (m, 2H), 5.46-5.67 (m, 2H).
¹³C-NMR (50 MHz, CDCl₃): δ (ppm) = 18.7:18.9, 23.3, 24.8, 26.0, 26.3, 26.8, 27.6, 31.0, 64.4:65.3, 71.3:65.7, 72.8:73.0, 127.1:126.2, 135.4:134.3, 173.7.

(RS)-3-Methyl-1,4-dioxacyclopentadecan-2-on (x = 1, y = 8, R¹ = Me, R² = H): Zu einer Lösung aus 240 mg (1 mmol) (RS)-3-Methyl-1,4-dioxa-(E/Z)-6-cyclopentadecen-2-on und 10 ml Isopropanol gibt man 12 mg Pd/C. Anschließend wird bei Raumtemperatur und Normaldruck hydriert. Nach 3 h wird über Celite abgesaugt und am Rotationsverdampfer das Lösungsmittel entfernt. Nach Flashchromatographie (Cyclohexan/EtOAc = 30:1, R_{f} = 0,28) an Kieselgel erhält man 177 mg (74%) als farbloses Öl.

Geruch: Moschus, süß-blumig, ambriert, erogen, animalisch, an Ambrett Moschus und Nitromoschus erinnernd.
¹H-NMR (200 MHz, CDCl₃): δ (ppm) = 1.20-1.50 (m, 14H), 1.40 (d, J = 6.8 Hz, 3H), 1.57-1.80 (m, 4H), 3.50 (m, 2H), 3.99 (q, J = 6.8 Hz, 1H), 4.20 (ddd, J = 6.1, 4.7, 1.3 Hz, 2H).
¹³C-NMR (50 MHz, CDCl₃): δ (ppm) = 18.6, 23.3, 24.5, 24.9, 25.2, 26.2, 26.3, 26.5, 27.7, 28.2, 64.9, 70.4, 75.6, 174.1.

### Beispiel 2.2:

### (S)-(-)-3-Methyl-1,4-dioxacyclopentadecan-2-on

(S)-(-)-2-Allyloxy-propionsäureethylester (x = 1, R¹ = Me, R² = H, R³ = Et): Man legt 7,08 g (60 mmol) (S)-(-)-Milchsäureethylester in 120 ml Cyclohexan vor und fügt 25,0 g (120 mmol) Allyltrichloracetimidat, gelöst in 30 ml Cyclohexan, hinzu. Abschließend werden 0,55 ml (6 mmol) Trifluormethansulfonsäure zugegeben und 16 h gerührt. Nach beendeter Reaktion wird das ausgefallene Trichloracetamid abgesaugt und mit Cyclohexan gewaschen. Das Filtrat wird mit gesättigter NaHCO₃-Lösung gewaschen, wonach die organische Phase über Na₂SO₄ getrocknet, abfiltriert und einrotiert wird. Nach flashchromatographischer Reinigung (Cyclohexan/EtOAc = 10:1; R_{f} = 0,27) erhält man 6,9 g (72%) einer farblosen Flüssigkeit.

Drehwert: [α]_{D}²⁰ = -70,1°.
Enantiomerenreinheit: ee = 95,2%.

(S)-(-)-2-Allyloxy-propionsäuredec-9-enylester (x = 1, y = 8, R¹ = Me, R² = H): Man legt 3,0 g (19 mmol) 2-Allyloxypropionsäureethylester in 4,5 g (28,5 mmol) 9-Decen-1-ol vor und fügt 0,56 g (2 mmol) Ti(OiPr)₄ zu. Jetzt wird auf 80°C erhitzt und bei einem Vakuum von 400 mbar 5 h gerührt. Anschließend gibt man einige Tropfen Wasser hinzu und reinigt das Rohprodukt mittels Flashchromatographie (Cyclohexan/EtOAc = 25:1; R_{f} = 0,21), so daß 4,1 g (81%) eines farblosen Öls erhalten wurden.

Drehwert: [α]_{D}²⁰ = -46,6°.
Enantiomerenreinheit: ee = 94,8%.

Die Ringschluß Olefinmetathese und nachfolgende Hydrierung wurden analog der unter 2.1 beschriebenen Vorschriften durchgeführt. Somit sind an dieser Stelle nur der Drehwert und die Enantiomerenreinheit aufgeführt:
(S)-(-)-3-Methyl-1,4-dioxa-(E/Z)-6-cyclopentadecen-2-on (x = 1, y = 8, R¹ = Me, R² = H):
   Geruch: schwach Moschus, süß-blumig, erogen, schwächer als das Enantiomerengemisch.
      Drehwert: [α]_{D}²⁰ = -20,0°.
      Enantiomerenreinheit: Die Enantiomerenreinheit für die beiden E/Z-Isomeren ist aufgrund von Peaküberlagerungen nicht bestimmbar.
(S)-(-)-3-Methyl-1,4-dioxacyclopentadecan-2-on (x = 1, y = 8, R¹ = Me, R² = H):
   Geruch: Moschus, süß-blumig, ambriert, erogen, animalisch, an Ambrett Moschus und Nitromoschus erinnernd, schwächer als das Enantiomerengemisch.
      Drehwert: [α]_{D}²⁰ = -23,0°.
      Enantiomerenreinheit: ee = 95,2%.

Die folgenden Antipoden sind in entsprechender Weise zugänglich. Von ihnen sind daher nur der Drehwert und die Enantiomerenreinheit aufgeführt:

### Beispiel 2.3:

### (R)-(+)-3-Methyl-1,4-dioxacyclopentadecan-2-on

R)-(+)-2-Allyloxy-propionsäureisobutylester (x = 1, R¹ = Me, R² = H, R³ = i-Bu):
Drehwert: Keine Drehwertbestimmung, da die Verbindung als Rohprodukt weiter eingesetzt wurde.
   Enantiomerenreinheit: ee = 99%.

(R)-(+)-2-Allyloxy-propionsäuredec-9-enylester (x = 1, y = 8, R¹ = Me, R² = H):
Drehwert: Keine Drehwertbestimmung, da die Verbindung als Rohprodukt weiter eingesetzt wurde.
   Enantiomerenreinheit: ee = 99%.

(R)-(+)-3-Methyl-1,4-dioxa-(E/Z)-6-cyclopentadecen-2-on (x = 1, y = 8, R¹ = Me, R² = H):
Geruch: Moschus, süß-blumig, ambriert, erogen, an Ambrett Moschus erinnernd, moschushafter und erogener als das (S)-(-)-Enantiomer.
   Drehwert: [α]_{D}²⁰ = +20,0°.
   Enantiomerenreinheit: Die Enantiomerenreinheit für die beiden E/Z-Isomeren ist aufgrund von Peaküberlagerungen nicht bestimmbar.

(R)-(+)-3-Methyl-1,4-dioxacyclopentadecan-2-on (x = 1, y = 8, R¹ = Me, R² = H):
Geruch: Moschus, süß-blumig, ambriert, erogen, animalisch, an Ambrett Moschus und Nitromoschus erinnernd, stärker als das (S)-(-)-Enantiomer.
   Drehwert: [α]_{D}²⁰ = +20,4°.
   Enantiomerenreinheit: ee = 99%.

Die folgenden Verbindungen der Beispiele 3.1, 3.2, 3.3 sind analog den unter Beispiel 2.1 beschriebenen Vorschriften hergestellt worden. Von Ihnen sind daher nur die spektroskopischen Daten aufgeführt:

### Beispiel 3.1:

### (RS)-3-Methyl-1,4-dioxacyclohexadecan-2-on

(RS)-2-Allyloxy-propionsäureundec-10-enylester (x = 1, y = 9, R¹ = Me, R² = H):
¹H-NMR (200 MHz, CDCl₃): δ (ppm) = 1.25-1.44 (m, 12H), 1.42 (d, J = 6.9 Hz, 3H), 1.57-1.76 (m, 2H), 1.98-2.11 (m, 2H), 3.94 (ddd, J = 12.5, 6.1, 1.3 Hz, 2H), 4.01 (q, J = 6.9 Hz, 1H), 4.13 (m, 2H), 4.93 (ddd, J = 10.2, 2.2, 1.2 Hz, 1H), 4.99 (ddd, J = 17.1, 2.2, 1.5 Hz, 1H), 5.20 (ddd, J = 10.3, 1.7, 1.1 Hz, 1H), 5.29 (dq, J = 17.3, 1.7 Hz, 1H), 5.81 (ddd. J = 17.1, 10.2, 6.7 Hz, 1H), 5.95 (ddd, J = 17.3, 10.3, 6.2 Hz, 1H).
   ¹³C-NMR (50 MHz, CDCl₃): δ (ppm) = 18.7, 25.8, 28.5, 28.9, 29.0, 29.1, 29.3, 29.4, 33.8, 64.9, 71.0, 74.0, 114.1, 117.6, 134.1, 139.1, 173.4.
(RS)-3-Methyl-1,4-dioxa-(E/Z)-6-cyclohexadecen-2-on (x = 1, y = 9, R¹ = Me, R² = H):
   Geruch: Moschus, süß-holzig, ambriert, erogen, animalisch, an Ambrett Moschus erinnernd.
      Angabe der Isomerie: Überschuß-:Unterschußisomer
      ¹H-NMR (200 MHz, CDCl₃): δ (ppm) = 1.22-1.53 (m, 12 H), 1.40:1.42 (d, J = 6.8:6.8 Hz, 3H), 1.65 (m, 2H), 2.11:2.05 (m, 2H), 3.96-4.36 (m, 4H), 4.10:4.02 (q, J = 6.8:6.8 Hz, 1H), 5.48-5.69 (m, 2H).
      ¹³C-NMR (50 MHz, CDCl₃): δ (ppm) = 18.2:18.8, 24.9, 25.4, 26.1, 26.3, 27.1, 27.5, 28.0, 31.3, 64.6:64.7, 69.8:66.2, 71.5:74.1, 126.4:125.9, 135.7:133.6, 173.4.
(RS)-3-Methyl-1,4-dioxacyclohexadecan-2-on (x = 1, y = 9, R¹ = Me, R² = H):
   Geruch: Moschus, süß-holzig, ambriert, erogen, animalisch, an Ambrett Moschus und Nitromoschus erinnernd.
      ¹H-NMR (200 MHz, CDCl₃): δ (ppm) = 1.25-1.50 (m, 16H), 1.40 (d, J = 6.7 Hz, 3H), 1.53-1.76 (m, 4H), 3.31-3.63 (m, 2H), 3.99 (q, J = 6.7 Hz, 1H), 4.21 (dd, J = 5.6, 5.0 Hz, 2H)
      ¹³C-NMR (50 MHz, CDCl₃): δ (ppm) = 18.1, 24.2, 24.6, 25.4, 26.5, 26.6, 26.7, 26.8, 26.9, 28.4, 28.7, 64.8, 69.9, 75.5, 173.6.

### Beispiel 3.2:

### (S)-(-)-3-Methyl-1,4-dioxacyclohexadecan-2-on

(S)-(-)-2-Allyloxy-propionsäureundec-10-enylester (x = 1, y = 9, R¹ = Me, R² = H):
Drehwert: [α]_{D}²⁰ = -36,0°.
   Enantiomerenreinheit: ee = 94,6%.
(S)-(-)-3-Methyl-1,4-dioxa-(E/Z)-6-cyclohexadecen-2-on (x = 1, y = 9, R¹ = Me, R² =H):
   Geruch: schwach Moschus, süß-holzig, erogen.
      Drehwert: [α]_{D}²⁰=-23,0°.
      Enantiomerenreinheit: Die Enantiomerenreinheit für die beiden E/Z-Isomeren ist aufgrund von Peaküberlagerungen nicht bestimmbar.
(S)-(-)-3-Methyl-1,4-dioxacyclohexadecan-2-on (x = 1, y = 9, R¹ = Me, R² = H):
   Geruch: Moschus, süß-holzig, ambriert, erogen, animalisch, an Ambrett Moschus und Nitromoschus erinnernd, schwächer als das Enantiomerengemisch.
      Drehwert: [α]_{D}²⁰= -16,0°.
      Enantiomerenreinheit: ee = 95,2%.

### Beispiel 3.3:

### (R)-(+)-3-Methyl-1,4-dioxacyclohexadecan-2-on

(R)-(+)-2-Allyloxy-propionsäureundec-10-enylester (x = 1, y = 9, R¹ = Me, R² = H):
Drehwert: Keine Drehwertbestimmung, da die Verbindung als Rohprodukt weiter eingesetzt wurde.
   Enantiomerenreinheit: ee = 99%.
(R)-(+)-3-Methyl-1,4-dioxa-(E/Z)-6-cyclohexadecen-2-on (x = 1, y = 9, R¹ = Me, R² = H):
   Geruch: Moschus, süß-holzig, ambriert, erogen, animalisch, an Ambrett Moschus erinnernd, stärker als das Enantiomerengemisch.
      Drehwert: [α]_{D}²⁰ = +28,2°.
      Enantiomerenreinheit: Die Enantiomerenreinheit für die beiden E/Z-Isomeren ist aufgrund von Peaküberlagerungen nicht bestimmbar.
(R)-(+)-3-Methyl-1,4-dioxacyclohexadecan-2-on (x = 1, y = 9, R¹ = Me, R² = H):
   Geruch: Moschus, süß-holzig, ambriert, erogen, animalisch, an Ambrett Moschus und Nitromoschus erinnernd, stärker als das (S)-(-)-Enantiomer.
      Drehwert: [α]_{D}²⁰ +15,40.
      Enantiomerenreinheit: ee = 99%.

### Beispiel 4:

### 1,4-Dioxacyclopentadecan-2-on

2-Allyloxy-essigsäure (x = 1, Y = OH, R¹, R² = H): Zu einer Suspension aus 2,4 g (60 mmol) NaH in 30 ml THF werden 1,8 g (30 mmol) Allylalkohol, gelöst in 10 ml THF, gegeben. Anschließend wird 3,4 g (25 mmol) Bromessigsäure, gelöst in 20 ml THF, zugetropft. Jetzt wird 6 h unter Rückfluß erhitzt. Nach dieser Zeit läßt man abkühlen und quencht die Reaktion mit 40 ml 2N HCI. Die wässrige Phase wird noch dreimal mit EtOAc extrahiert. Die vereinigten organischen Phasen werden über Na₂SO₄ getrocknet, abfiltriert und am Rotationsverdampfer zur Trockne eingeengt. Man erhält 3,8 g Rohprodukt (GC-Reinheit 90,3%), welches ohne weitere Reinigung in die nächsten Reaktionen eingesetzt wird.

2-Allyloxy-essigsäuredec-9-enylester (x = 1, y = 8, R¹, R² = H): Man legt 2,5 g rohe 2-Allyloxyessigsäure in 40 ml Toluol vor und fugt 4,7 g (30 mmol) 9-Decen-1-ol sowie 0,38 g (2,0 mmol) p-Toluolsulfonsäure hinzu. Jetzt wird am Wasserabscheider erhitzt bis sich keine sichtbaren Mengen Wasser mehr abscheiden. Anschließend läßt man abkühlen, wäscht die organische Phase einmal mit gesättigter NaHCO₃-Lösung, trocknet über Na₂SO₄, filtriert ab und befreit das Produkt vom Lösungsmittel. Nach Flashchromatographie (Cyclohexan/EtOAc = 20:1, R_{f} = 0,29) erhält man 3,1 g (62%) 2-Allyloxy-essigsäuredec-9-enylester als farbloses Öl.

¹H-NMR (200 MHz, CDCl₃): δ (ppm) = 1.20-1.45 (m, 10H), 1.55-1.73 (m, 2H), 1.96-2.02 (m, 2H), 4.08 (s, 2H), 4.10 (dt, J = 5.8, 1.2 Hz, 2H), 4.16 (t, J = 6.7 Hz, 2H), 4.93 (ddd, J = 10.3, 2.2, 1.2 Hz, 1H), 4.99 (ddd, J = 17.3, 2.2, 1.5 Hz, 1H), 5.24 (ddd, J = 10.3, 1.7, 1.2 Hz, 1H), 5.31 (dq, J = 17.3, 1.7 Hz, 1H), 5.81 (ddd, J = 17.3, 10.3,6.6 Hz, 1H), 5.93 (ddd, J = 17.3, 10.3, 5.8 Hz, 1H).
¹³C-NMR (50 MHz, CDCl₃): δ (ppm) = 25.8, 28.5, 28.8, 29.0, 29.1, 29.3, 33.7, 64.9, 67.1, 72.3, 114.1, 118.2, 133.7, 139.1, 170.4.

Die Ringschluß Olefinmetathese, wie auch die nachfolgende Hydrierung werden analog der unter Beispiel 2.1 beschriebenen Synthesvorschriften durchgerührt, so daß an dieser Stelle nur die spektroskopischen Daten aufgeführt werden:

1,4-Dioxa-(E/Z)-6-cyclopentadecen-2-on (x = 1, y = 8, R¹, R² = H):
Geruch: Moschus, metallisch, an heißes Bügeleisen erinnernd.
   Angabe der Isomerie: Überschuß-:Unterschußisomer
   ¹H-NMR (200 MHz, CDCl₃): δ (ppm) = 1.20-1.50 (m, 10H), 1.55-1.80 (m, 2H), 2.0-2.16 (m, 2H), 4.09 (s, 2H), 4.11-4.33 (m, 4H), 5.43-5.75 (m, 2H).
   ¹³C-NMR (50 MHz, CDCl₃): δ (ppm) = 23.1, 25.2, 26.1, 26.5, 27.3, 27.5, 31.2, 64.2:65.3, 65.7:66.1, 72.6, 126.6:125.5, 136.6:135.6, 171.2.
1,4-Dioxacyclopentadecan-2-on (x = 1, y = 8, R¹, R² = H):
   Geruch: Moschus, süß-blumig, erogen, an Moschus Ambrett erinnernd.
      ¹H-NMR (200 MHz, CDCl₃): δ (ppm) =1.30-1.51 (m, 14H), 1.60-1.80 (m, 4H), 3.52 (t, J = 6.6 Hz, 2H), 4.11 (s, 2H), 4.22 (dd, J = 5.1, 4.4 Hz, 2H).
      ¹³C-NMR (50 MHz, CDCl₃): δ (ppm) = 23.7, 24.7, 25.3, 25.5, 26.2, 26.6, 26.9, 27.7, 28.0, 65.3, 69.3, 71.9, 171.3.

Die folgenden Verbindungen sind in entsprechender Weise zugänglich. Von ihnen sind daher nur die spektroskopischen Daten aufgeführt:

### Beispiel 5:

### 1,4-Dioxacyclohexadecan-2-on

2-Allyloxy-essigsäureundec-10-enylester (x = 1, y = 9, R¹, R² = H):
¹H-NMR (200 MHz, CDCl₃): δ (ppm) = 1.20-1.45 (m, 12H), 1.55-1.73 (m, 2H), 1.96-2.11 (m, 2H), 4.08 (s, 2H), 4.10 (dt, J = 5.8, 1.4 Hz, 2H), 4.16 (t, J = 6.7 Hz, 2H), 4.93 (ddd, J = 10.2, 2.2, 1.3 Hz, 1H), 4.99 (ddd, J = 17.1, 2.2, 1.4 Hz, 1H), 5.24 (ddd, J = 10.2, 1.5, 1.2 Hz, 1H), 5.31 (dq, J = 17.2, 1.5 Hz, 1H), 5.81 (ddd, J = 17.2, 10.2, 6.7 Hz, 1H), 5.93 (ddd, J = 17.2, 10.2, 5.8 Hz, 1H).
   ¹³C-NMR (50 MHz, CDCl₃): δ (ppm) = 25.8, 28.5, 28.9, 29.0, 29.2, 29.3, 29.4, 33.8, 65.0, 67.1, 72.3, 114.1, 118.1, 133.7, 139.1, 170.4.
1,4-Dioxa-(E/Z)-6-cyclohexadecen-2-on (x = 1, y = 9, R¹, R² = H):
   Geruch: Moschus, holzig, technisch an heißes Bügeleisen erinnernd.
      Angabe der Isomerie: Überschuß-:Unterschußisomer
      ¹H-NMR (200 MHz, CDCl₃): δ (ppm) = 1.20-1.50 (m, 12H), 1.61-1.78 (m, 2H), 2.00-2.20 (m, 2H), 4.07-4.14 (m, 2H), 4.10:4.11 (s, 2H), 4.18-4.29 (m, 2H), 5.41-5.76 (m, 2H).
      ¹³C-NMR (50 MHz, CDCl₃): δ (ppm) = 25.3, 25.9, 26.4, 26.9, 27.6, 27.7, 27.9, 31.6, 64.7:66.7, 64.8:66.8, 70.8, 125.8:125.2, 137.3:135.1, 170.5.
1,4-Dioxacyclohexadecan-2-on (x = 1, y = 9, R¹, R² = H):
   Geruch: Moschus, ambriert, erogen, animalisch, an Moschus-Tinktur, Moschus Ambrett und Nitromoschus erinnernd.
      ¹H-NMR (200 MHz, CDCl₃): δ (ppm) = 1.25-1.51 (m, 16H), 1.55-1.75 (m, 2H), 3.53 (t, J = 5.8 Hz, 2H), 4.08 (s, 2H), 4.24 (dd, J = 5.2, 5.1 Hz, 2H).
      ¹³C-NMR (50 MHz, CDCl₃): δ (ppm) = 24.2, 24.3, 25.3, 25.4, 26.5, 26.6, 26.8, 26.9, 28.1, 28.5, 64.8, 69.0, 71.2, 170.5.

### Beispiel 6:

### 1,4-Dioxa-(E/Z)-7-cyclohexadecen-2-on

2-(3-Butenyloxy)-essigsäuredec-9-enylester (x = 2, y = 8, R¹, R² = H):
¹H-NMR (200 MHz, CDCl₃): δ (ppm) = 1.20-1.41 (m, 10H), 1.57-1.71 (m, 2H), 1.98-2.12 (m, 2H), 2.40 (qd, J = 6.7, 1.4 Hz, 2H), 3.60 (t, J = 6.6 Hz, 2H), 4.09 (s, 2H), 4.15 (t, J = 6.8 Hz, 2H), 4.92 (ddd, J = 10.3, 2.2, 1.3 Hz, 1H), 4.95 (ddd, J = 16.4, 2.2, 1.4 Hz, 1H), 5.11 (ddd, J = 10.3, 1.5, 1.2 Hz, 1H), 5.18 (dq, J = 16.4, 1.4 Hz, 1H), 5.75 (ddd, J = 16.4, 10.3, 6.6 Hz, 1H), 5.93 (ddd, J = 16.4, 10.3, 5.8 Hz, 1H).
   ¹³C-NMR (50 MHz, CDCl₃): δ (ppm) = 25.8, 28.6, 28.9, 29.0, 29.2, 29.3, 33.8, 34.0, 65.0, 68.3, 71.0, 114.2, 116.7, 134.8, 139.1, 170.6.
1,4-Dioxa-(E/Z)-7-cyclohexadecen-2-on (x = 2, y = 8, R¹, R² = H):
   Geruch: Moschus, erogen, süß-holzig, metallisch, an heißes Bügeleisen erinnernd.
   Angabe der Isomerie: Überschuß-:Unterschußisomer
   ¹H-NMR (200 MHz, CDCl₃): δ (ppm) = 1.20-1.50 (m, 10H), 1.59-1.73 (m, 2H), 1.98-2.12 (m, 2H), 2.25-2.48 (m, 2H), 3.44-3.62 (m, 2H), 4.09:4.11 (s, 2H), 4.18-4.30 (m, 2H), 5.40-5.50 (m, 2H).
   ¹³C-NMR (50 MHz, CDCl₃): δ (ppm) = 23.8, 25.3, 26.7, 26.8, 27.5, 27.6, 31.6, 33.1, 65.0:64.2, 69.3:69.2, 71.3:71.4, 127.5:125.0, 132.2, 170.4.

### Beispiel 7:

### 1,4-Dioxa (E/Z)-7-cycloheptadecen-2-on

2-(3-Butenyloxy)-essigsäureundec-10-enylester (x = 2, y = 9, R¹, R² = H):
¹H-NMR (200 MHz, CDCl₃): δ (ppm) = 1.20-1.48 (m, 12H), 1.55-1.74 (m, 2H), 1.97-2.12 (m, 2H), 2.40 (qd, J = 6.9, 1.2 Hz, 2H), 3.60 (t, J = 6.7 Hz, 2H), 4.09 (s, 2H), 4.15 (t, J = 6.8 Hz, 2H), 4.92 (ddd, J = 10.2, 2.2, 1.4 Hz, 1H), 4.96 (ddd, J = 16.3, 2.2, 1.4 Hz, 1H), 5.08 (ddd, J = 10.3, 1.4, 1.2 Hz, 1H), 5.17 (dq, J = 16.5, 1.4 Hz, 1H), 5.75 (ddd, J=16.3, 10.3, 6.5 Hz, 1H), 5.93 (ddd, J = 16.5, 10.3, 5.7 Hz, 1H).
   ¹³C-NMR (50 MHz, CDCl₃): δ (ppm) = 25.8, 28.5, 28.8, 29.0, 29.1, 29.3, 29.4, 33.8, 34.0, 64.9, 68.3, 71.0, 114.1, 116.6, 134.7, 139.1, 170.5.
1,4-Dioxa-(E/Z)-7-cycloheptadecen-2-on (x = 2, y = 9, R¹, R² = H):
   Geruch: Moschus, erogen, süß-holzig.
      Angabe der Isomerie: Überschuß-:Unterschußisomer
      ¹H-NMR (200 MHz, CDCl₃): δ (ppm) = 1.21-1.51 (m, 12H), 1.60-1.78 (m, 2H), 2.00-2.18 (m, 2H), 2.26-2.50 (m, 2H), 3.48-3.64 (m, 2H), 4.12 (s, 2H), 4.19-4.29 (m, 2H), 5.34-5.51 (m, 2H).
      ¹³C-NMR (50 MHz, CDCl₃): δ (ppm) = 26.9, 27.6, 28.0, 28.1, 28.2, 28.4, 28.5, 31.5, 32.9, 65.4:65.2, 69.2:68.9, 71.6, 126.7:124.4, 132.6, 170.7

### Beispiel 8:

### (RS)-3-Methyl-1,4-dioxa-(E/Z)-7-cyclohexadecen-2-on

2-(3-Butenyloxy)-propionsäuredec-9-enylester (x = 2, y = 8, R¹ = Me, R² = H):
¹H-NMR (200 MHz, CDCl₃): δ (ppm) = 1.26-1.44 (m, 10H), 1.40 (d, J = 6.9 Hz, 3H), 1.60-1.70 (m, 2H), 1.98-2.07 (m, 2H), 2.37 (qt, J = 6.9, 1.5 Hz, 2H), 3.42 (dt, J = 8.5, 6.8 Hz, 1H), 3.64 (dt, J = 8.5, 6.8 Hz, 1H), 3.81 (q, J = 6.8 Hz, 1H), 4.14 (td, J = 6.8, 2.6 Hz, 2H), 4.90 (ddd, J = 10.2, 2.2, 1.4 Hz, 1H), 4.95 (ddd, J = 16.3, 2.2, 1.4 Hz, 1H), 5.07 (ddd, J = 10.3, 1.4, 1.2 Hz, 1H), 5.15 (dq, J = 16.5, 1.4 Hz, 1H), 5.74 (ddd, J = 16.3, 10.3, 6.5 Hz, 1H), 5.91 (ddd, J = 16.5, 10.3, 5.7 Hz, 1H).
   ¹³C-NMR (50 MHz, CDCl₃): δ (ppm) = 18.7, 25.8, 28.5, 28.8, 28.9, 29.1, 29.3, 33.7, 34.1, 64.8, 69.4, 74.9, 114.0, 116.4, 134.7, 139.0, 173.3.
(RS)-3-Methyl-1,4-dioxa-(E/Z)-7-cyclohexadecen-2-on (x = 2, y = 8, R¹ = Me, R² = H):
   Geruch: Moschus, holzig, metallisch, an heißes Bügeleisen erinnernd.
      Angabe der Isomerie: Überschuß-:Unterschußisomer
      ¹H-NMR (200 MHz, CDCl₃): δ (ppm) = 1.22-1.42 (m, 10H), 1.40:1.42 (d, J = 6.8 Hz, 3H), 1.55-1.72 (m, 2H), 1.96-2.09 (m, 2H), 2.19-2.38 (m,2H), 3.37-3.57 (m, 2H), 3.98:4.00 (q, J = 6.8 Hz, 1H), 4.10-4.30 (m, 2H), 5.39-5.49 (m, 2H).
      ¹³C-NMR (50 MHz, CDCl₃): δ (ppm) = 18.2, 24.5, 24.9, 26.8, 27.1, 28.0, 28.1, 31.1, 33.1, 65.0:64.1, 70.4:70.1, 76.8, 127.5:124.5, 132.1, 173.4.

### Beispiel 9:

### (RS)-3-Methyl-1,4-dioxacycloheptadecan-2-on (nitch beansprucht)

2-(3-Butenyloxy)-propionsäureundec-10-enylester (x = 2, y = 9, R¹ = Me, R² = H):
¹H-NMR (200 MHz, CDCl₃): δ (ppm) = 1.24-1.42 (m, 12H), 1.40 (d, J = 6.8 Hz, 3H), 1.58-1.70 (m, 2H), 1.97-2.10 (m, 2H), 2.37 (qt, J = 6.9, 1.4 Hz, 2H), 3.42 (dt, J = 9.0, 6.9 Hz, 1H), 3.64 (dt, J = 9.0, 6.9 Hz, 1H), 3.96 (q, J = 6.8 Hz, 1H), 4.15 (td, J = 6.5, 2.6 Hz, 2H), 4.91 (ddd, J = 10.1, 2.2, 1.4 Hz, 1H), 4.95 (ddd, J = 17.0, 2.2, 1.4 Hz, 1H), 5.07 (ddd, J = 10.2, 1.4, 1.2 Hz, 1H), 5.15 (dq, J = 17.2, 1.4 Hz, 1H), 5.74 (ddd, J = 17.0, 10.1, 6.5 Hz, 1H), 5.91 (ddd, J = 17.2, 10.2, 5.7 Hz, 1H).
   ¹³C-NMR (50 MHz, CDCl₃): δ (ppm) = 18.6, 25.8, 28.5, 28.8, 29.0, 29.1, 29.3, 29.4, 33.7, 34.1, 64.8, 69.4, 74.9, 114.0, 116.4, 134.7, 139.0, 173.3.
(RS)-3-Methyl-1,4-dioxa-(E/Z)-7-cycloheptadecen-2-on (x = 2, y = 9, R¹ = Me, R² = H):
   Geruch: Moschus, erogen, süß-holzig.
      ¹H-NMR (200 MHz, CDCl₃): δ (ppm) = 1.20-1.44 (m, 12H), 1.40:1.42 (d, J = 6.8 Hz, 3H), 1.60-1.75 (m, 2H), 1.98-2.10 (m, 2H), 2.25-2.37 (m,2H), 3.34-3.60 (m, 2H), 4.00:3.98 (q, J = 6.8 Hz, 1H), 4.15-4.28 (m, 2H), 5.40-5.50 (m, 2H).
      ¹³C-NMR (50 MHz, CDCl₃): δ (ppm) = 18.5:18.8, 25.8, 27.1, 27.5, 27.7, 27.8, 28.6, 28.7, 31.6, 32.9, 65.0, 70.5:70.2, 75.5:75.7, 126.0:124.0, 132.9:132.5, 173.6
(RS)-3-Methyl-1,4-dioxacycloheptadecan-2-on (x = 2, y = 9, R¹ = Me, R² = H):
   Geruch: Moschus, süß-holzig, ambriert, erogen, animalisch, an Ambrett Moschus erinnernd.
      ¹H-NMR (200 MHz, CDCl₃): δ (ppm) = 1.26-1.44 (m, 18H), 1.40 (d, J = 6.8 Hz, 3H), 1.55-1.75 (m, 4H), 3.38-3.60 (m, 2H), 3.79 (q, J = 6.8 Hz, 1H), 4.10-4.30 (m, 2H).
      ¹³C-NMR (50 MHz, CDCl₃): δ (ppm) = 18.6, 24.7, 25.3, 26.1, 26.4, 26.6, 26.7, 27.1, 27.3, 27.4, 28.6, 28.7, 64.9, 70.3, 75.5, 173.7.

### Beispiel 10:

Das vorliegende Parfümöl dient zur Parfümierung vielerlei kosmetischer Produkte.

### Zusammensetzung:

| Ingredienzien | Gewichtsteile |
|---|---|
| 1. Citrophoral Base (H&R) | 5,0 |
| 2. Aldehyd C10 10% in BA | 5,0 |
| 3. Aldehyd C11 MOA 10% in BA | 3,0 |
| 4. Farenal (H&R) | 3,0 |
| 5. Aldehyd C11 10% in IPM | 5,0 |
| 6. Citroxal 50% in DEP | 2,0 |
| 7. trans Hex-2-enol 10% in BA | 2,0 |
| 8. Vertocitral (H&R) | 1,0 |
| 9. Linalylaceta | 45,0 |
| 10. Citrylal (H&R) | 5,0 |
| 11. Mandarinal (Firmenich) | 4,0 |
| 12. Lilial (Givaudan Roure) | 75,0 |
| 13. Lyral (IFF) | 75,0 |
| 14. Profarnesol (H&R) | 5,0 |
| 15. Nerolidol | 5,0 |
| 16. Linalool | 45,0 |
| 17. Geranimöl afrikanisch | 5,0 |
| 18. Phenylethyalkohol | 75,0 |
| 19. Geraniol | 15,0 |
| 20. Nerol | 10,0 |
| 21. Hexylzimtaldehyd alpha | 50,0 |
| 22. Methyldihydrojasmonat | 15,0 |
| 23. Benzylsalicylat | 100,0 |
| 24. trans,cis-2-Nonadienol 0,1% in IPM | 5,0 |
| 25. Allylionon (Givaudan Roure) | 3,0 |
| 26. Isomethylionon gamma | 75,0 |
| 27. Eugenol | 7,0 |
| 28. Cedrylacetat | 40,0 |
| 29. Sandolen (H&R) | 5,0 |
| 30. Citral | 5,0 |
| BA=Benzylalkohol; IPM= Isopropylmyristat; DEP=Diethylphtalat | |

Der Zusatz von
a) 355 Gewichtsteilen 3-Methyl-1,4-dioxacyclopentadecan-2-on (Summe 1000 Gewichtsteile) führt zu einer deutlich wahrnehmbaren Harmonisierung der frischen Kopfnote mit der rosig-blumigen Herznote. Darüber hinaus werden mit 3-Methyl-1,4-dioxacyclopentadecan-2-on an Nitromoschus erinnernde Effekte erzielt und die feine erogene Moschusnote verleiht der vorliegenden Komposition eine hervorragende Strahlung und gesteigerte Haftung. Hierbei setzt sich besonders der wertvolle Charakter von 3-Methyl-1,4-dioxacyclopentadecan-2-on im Vergleich zu Kompositionen mit konventionellen Moschusriechstoffen durch.
b) 55 Gewichtsteilen 3-Methyl-1,4-dioxacyclohexadecan-2-on (Summe 700 Gewichtsteile) verleiht der Komposition eine animalische Moschusnote, die mit existierenden Moschusriechstoffen nicht erreicht wird. Weiterhin gewinnt die gesamte Komposition an Fülle und erscheint wertvoller.

## Patentansprüche

1. 1,4-Dioxacycloalkan-2-one und 1,4-Dioxacycloalken-2-one der Formel 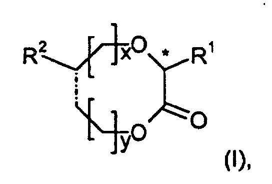 worin
die gestrichelte Bindung eine Einfach-oder E/Z-Doppelbindung ist,
wobei für den Fall, daß eine Doppelbindung im Ring vorliegt die Verbindungen in der E und Z-Form vorliegen können, und
Verbindungen mit einem chiralen Zentrum sowohl in der (R)- oder (S)-Form als auch als Enantiomerengemisch vorliegen können,
R¹ und R² gleich oder verschieden sind und Wasserstoff oder Niederalkyl ten,
x für eine gesättigte Alkylenkette mit 1 bis 4 Kohlenstoffatomen und
y für eine gesättigte Alkylenkette mit 4 bis 10 Kohlenstoffatomen steht,
ausgewählt aus der Gruppe, die aus
3-Methyl-1,4-dioxa-(E/Z)-6-cyclopentadecen-2-on (x = 1, y = 8, R¹ = Me, R² = H)
3-Methyl-1,4-dioxacyclopentadecan-2-on (x = 1, y = 8, R¹ = Me, R² = H)
1,4-Dioxa-(E/Z)-6-cyclopentadecen-2-on (x = 1, y = 8, R¹, R² = H)
1,4-Dioxacyclopentadecan-2-on (x = 1, y = 8, R¹, R² = H)
3-Methyl-1,4-dioxa-(E/Z)-6-cyclohexadecen-2-on (x = 1, y = 9, R¹ = Me, R² = H)
3-Methyl-1,4-dioxacyclohexadecan-2-on (x = 1, y = 9, R¹ = Me, R² = H)
1,4-Dioxa-(E/Z)-6-cyclohexadecen-2-on (x = 1, y = 9, R¹, R² = H)
1,4-Dioxacyclohexadecan-2-on (x = 1, y = 9, R¹, R² = H)
1,4-Dioxa-(E/Z)-7-cyclohexadecen-2-on (x = 2, y = 8, R¹, R² = H) und
3-Methyl-1,4-dioxa-(E/Z)-7-cyclohexadecen-2-on (x = 2, y = 8, R¹ = Me, R² = H)
besteht.

2. Doppelt terminal ungesättigte 2-Alkenyloxycarbonsäurealkenylester der Formel 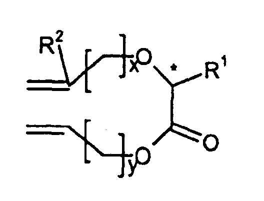 zur Herstellung einer Verbindung nach Anspruch 1,
wobei
Verbindungen mit einem chiralen Zentrum sowohl in der (R)- oder (S)-Form wie auch als Enantiomerengemisch vorliegen können,
R¹ und R² gleich oder verschieden sind und Wasserstoff oder Niederälkyl bedeuten,
x für eine gesättigte Alkylenkette mit 1 bis 4 Kohlenstoffatomen und
y für eine gesättigte Alkylenkette mit 4 bis 10 Kohlenstoffatomen steht,
wobei der 2-Alkenyloxycarbphsäurealkenylester ausgewählt ist aus der Gruppe, die aus Verbindungen besteht, für die gilt:
x = 1, y = 8, R¹ = Me, R² = H
x = 1, y = 8, R¹, R² = H
x = 1, y = 9, R¹ = Me, R² = H
x = 1, y = 9, R¹, R² = H
x = 2, y = 8, R¹, R² = H
oder
x = 2, y = 8, R¹ = Me, R² = H.

3. Verfahren zur Herstellung von makrocyclischen 1,4-Dioxacycloalkan-2-onen oder 1,4-Dioxacycloalken-2-onen nach Anspruch 1, wobei Verbindungen mit einem chiralen Zentrum als Enantiomerengemisch vorliegen,
**dadurch gekennzeichnet, dass** in 2-Position derivatisierbare Alkylcarbonsäuren oder deren Ester der Formel 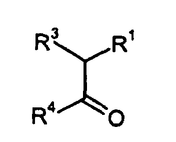 worin
R¹ die in Anspruch 1 für das jeweilige makrocyclische 1,4-Dioxacycloalkan-2-on oder 1,4-Dioxacycloalken-2-on genannte Bedeutung hat, und
R³ für OH, Cl oder Br und
R⁴ für OH, OMe oder ÖEt steht,
eingesetzt werden, die im 1. Schritt verethert, in einem 2. Schritt verestert und in einem 3. Schritt der Ring durch Olefimmetathese zu den ungesättigten 1,4-Dioxacycloalken-2-onen geschlossen wird, die dann gegebenenfalls in einem 4. Schritt zu den gesättigten 1,4-Dioxacycloalkan-2-onen hydriert werden.

4. Verfahren zur Herstellung von chiralen makrocyclischen 3-Alkyl-1,4-dioxacycloalkan-2-onen und 3-Alkyl-1,4-dioxacycloalken-2-onen der Formel 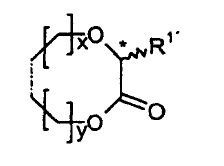 worin die gestrichelte Bindung eine Einfach oder E/Z-Doppelbindung ist,
wobei für den Fall, dass eine Doppelbindung im Ring vorliegt, die Verbindungen in der E und Z-Form vorliegen können,
Verbindungen mit einem chiralen Zentrum in der (R)- oder (S)-Form vorliegen,
R^{1'} die in Anspruch 1 für den Rest R¹ eines jeweiligen makrocyclischen 1,4-Dioxacycloalkan-2-ons oder 1,4-Dioxacydoalken-2-ons genannte Bedeutung hat, und
x die in Anspruch 1 für das jeweilige makrocyclische 1,4-Dioxacycloalkan-2-on oder 1,4-Dioxacycycloalken-2-on genannte Bedeutung hat
y die in Anspruch 1 für das jeweilige makrocyclische 1,4-Dioxacycloalkan-2-on oder 1,4-Dioxacycloalken-2-on genannte Bedeutung hat
**dadurch gekennzeichnet, dass** als Edukte (S)-2- oder (R)-2-Hydroxycarbonsäurealkylester der Formel 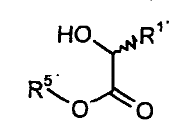 worin
R¹' die oben genannte Bedeutung hat, und
R⁵ für einen C1 bis C8 Alkylrest steht,
eingesetzt werden, die in einem 1. Schritt unter sauren, nicht racemisierenden Bedingungen verethert, in einem 2. Schritt unter Lewissäure-Katalyse umgeestert und in einem 3. Schritt der Ring durch Olefinmetathese zu den ungesättigten 3-Alkyl-1,4-dioxacycloalken-2-onen geschlossen wird, die dann gegebenenfalls in einem 4. Schritt zu den gesättigten 3-Alkyl-1,4-dioxacycloalkan-2-onen hydriert werden.

5. Riechstoffe, enthaltend 1,4-Dioxacycloalkan-2-one und 1,4-Dioxacycloalken-2-one nach Anspruch 1.

6. Riechstoffkompositionen, enthaltend 1,4-Dioxacycloalkan-2-one und 1,4-Dioxacycloalken-2-one nach Anspruch 1.

7. Riechstoffkompositionen nach Anspruch 6, enthaltend 1 bis 40 Gew.% makrocyclische 1,4-Dioxacycloalkan-2-one und 1,4-Dioxacycloalken-2-one nach Anspruch 1.

8. Verwendung von Riechstoffen nach Anspruch 5 in Riechstoffkompositionen mit Moschuscharakter.

## Claims

1. 1,4-Dioxacycloalkan-2-ones and 1,4-dioxacycloalken-2-ones of the formula 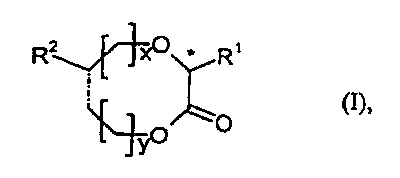 found, in which
the dashed bond is a single or E/Z double bond,
where in the case of a double bond being present in the ring, the compounds can be in the E and Z form, and
compounds with a chiral centre can be either in the (R) or (S) form, or else can be present as an enantiomer mixture,
R¹ and R² are identical or different and are hydrogen or lower alkyl,
x is a saturated alkylene chain having 1 to 4 carbon atoms and
y is a saturated alkylene chain having 4 to 10 carbon atoms,
selected from the group consisting of
3-Methyl-1,4-dioxa-(E/Z)-6-cyclopentadecen-2-one (x = 1, y = 8, R¹ = Me, R² = H)
3-Methyl-1,4-dioxacyclopentadecan-2-one (x = 1, y = 8, R¹ = Me, R² = H)
1,4-Dioxa-(E/Z)-6-cyclopentadecen-2-one (x = 1, y = 8, R¹, R² = H)
1,4-Dioxacyclopentadecan-2-one (x = 1, y = 8, R¹, R² = H)
3-Methyl-1,4-dioxa-(E/Z)-6-cyclohexadecen-2-one (x = 1, y = 9, R¹ = Me, R² = H)
3-Methyl-1,4-dioxacyclohexadecan-2-one (x = 1, y = 9, R¹ = Me, R² = H)
1,4-Dioxa-(E/Z)-6-cyclohexadecen-2-one (x = 1, y = 9, R¹, R² = H)
1,4-Dioxacyclohexadecan-2-one (x = 1, y = 9, R¹, R² = H)
1,4-Dioxa-(E/Z)-7-cyclohexadecen-2-one (x = 2, y = 8, R¹, R² = H) and
3-Methyl-1,4-dioxa-(E/Z)-7-cyclohexadecen-2-one (x = 2, y = 8, R¹ = Me, R² = H)

2. Doubly terminally unsaturated 2-alkenyloxycarboxylic alkenyl esters of the formula 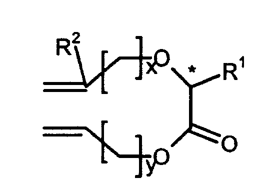 for preparing a compound according to Claim 1
in which
compounds with a chiral centre can be either in the (R) or (S) form, or else be present as an enantiomer mixture,
R¹ and R² are identical or different and are hydrogen or lower alkyl,
x is a saturated alkylene chain having 1 to 4 carbon atoms and
y is a saturated alkylene chain having 4 to 10 carbon atoms,
in which the 2-alkenyloxycarboxylic alkenyl ester is selected from the group consisting of compounds in which
x = 1, y = 8, R¹ = Me, R² = H
x = 1, y = 8, R⁴, R² = H
x = 1, y = 9, R¹ = Me, R² = H
x = 1, y = 9, R¹, R² = H
x = 2, y = 8, R¹, R² = H
or
x = 2, y = 8, R¹ = Me, R² = H.

3. Process for the preparation of macrocyclic 1,4-dioxacycloalkan-2-ones or 1,4-dioxacycloalken-2-ones according to Claim 1, in which compounds with a chiral centre are present as an enantiomer mixture, **characterized in that** alkylcarboxylic acids or esters thereof which can be derivatized in the 2 position and are of the formula 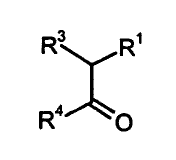 in which
R¹ has the meaning given in Claim 1 for the respective macrocyclic 1,4-dioxacycloalkan-2-one or 1,4-dioxacycloalken-2-one, and
R³ is OH, Cl or Br and
R⁴ is OH, OMe or OEt,
are used, which, in the 1st step, are etherified, in a 2nd step are esterified and in a 3rd step the ring is closed by olefin metathesis to the give the unsaturated 1,4-dioxacycloalken-2-ones, which are then optionally hydrogenated in a 4th step to give the saturated 1,4-dioxacycloalkan-2-ones.

4. Process for the preparation of chiral macrocyclic 3-alkyl-1,4-dioxacycloalkan-2-ones and 3-alkyl-1,4-dioxacycloalken-2-ones of the formula 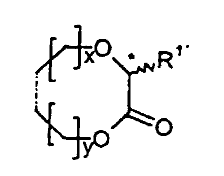 in which the dashed bond is a single or E/Z double bond,
where in the case of a double bond being present in the ring, the compounds can be in the E and Z form,
compounds with a chiral centre are present in the (R) or (S) form,
R¹ has the meaning given in Claim 1 for the radical R¹ of a respective macrocyclic 1,4-dioxacycloalkan-2-one or 1,4-dioxacycloalken-2-one and
x has the meaning given in Claim 1 for the respective macrocyclic 1,4-dioxacycloalkan-2-one or 1,4-dioxacycloalken-2-one
y has the meaning given in Claim 1 for the respective macrocyclic 1,4-dioxacycloalkan-2-one or 1,4-dioxacycloalken-2-one,
**characterized in that** the starting materials used are (S)-2- or (R)-2-hydroxycarboxylic alkyl esters of the formula 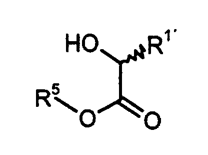 in which
R¹ has the meaning given above, and
R⁵ is a C₁ to C₈ alkyl radical,
which are etherified in a 1st step under acidic, nonracemizing conditions, are transesterified in a 2nd step under Lewis acid catalysis and in a 3rd step the ring is closed by olefin metathesis to give the unsaturated 3-alkyl-1,4-dioxacycloalken-2-ones, which are then optionally hydrogenated in a 4th step to give the saturated 3-alkyl-1,4-dioxacycloalkan-2-ones.

5. Fragrances comprising 1,4-dioxacycloalkan-2-ones and 1,4-dioxacycloalken-2-ones according to Claim 1.

6. Fragrance compositions comprising 1,4-dioxacycloalkan-2-ones and 1,4-dioxacycloalken-2-ones according to Claim 1.

7. Fragrance compositions according to Claim 6, comprising 1 to 40% by weight of macrocyclic 1,4-dioxacycloalkan-2-ones and 1,4-dioxacycloalken-2-ones according to Claim 1.

8. Use of fragrances according to Claim 5 in fragrance compositions with musk character.

## Revendications

1. 1,4-dioxacycloalcan-2-ones et 1,4-dioxacycloalcén-2-ones de formule 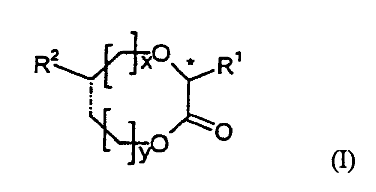 dans laquelle
la liaison en pointillés représente une liaison simple ou une double liaison E/Z, les composés pouvant se trouver sous forme E et Z dans le cas où il y a une double liaison dans le cycle, et les composés contenant un centre chiral pouvant se trouver aussi bien sous forme (R) ou (S) que sous forme d'un mélange d'énantiomères,
R¹ et R² sont identiques ou différents et représentent un atome d'hydrogène ou un groupe alkyle inférieur,
x représente une chaîne alkylène saturée de 1 à 4 atomes de carbone et
y représente une chaîne alkylène saturée de 4 à 10 atomes de carbone,
choisis dans le groupe constitué par
la 3-méthyl-1,4-dioxa-(E/Z)-6-cyclopentadécén-2-one (x = 1, y = 8, R¹ = Me, R² = H)
la 3-méthyl-1,4-dioxacyclopentadécan-2-one (x = 1, y = 8, R¹ = Me, R² = H)
la 1,4-dioxa-(E/Z)-6-cyclopentadécén-2-one (x = 1, y =8, R¹, R² = H)
la 1,4-dioxacyclopentadécan-2-one (x = 1, y = 8, R¹, R² = H)
la 3-méthyl-1,4-dioxa-(E/Z)-6-cyclohexadécén-2-one (x = 1, y = 9, R¹ = Me, R² = H)
la 3-méthyl-1,4-dioxacyclohexadécan-2-one (x = 1, y = 9, R¹ = Me, R² = H)
la l,4-dioxa-(E/Z)-6-cyclohexadécén-2-one (x = 1, y = 9, R¹, R² = H)
la 1,4-dioxacyclohexadécan-2-one (x = 1, y = 9, R¹, R² = H)
la 1,4-dioxa-(E/Z)-7-cyclohexadécén-2-one (x = 2, y = 8, R¹, R² = H) et
la 3-méthyl-1,4-dioxa-(E/Z)-7-cyclohexadécén-2-one (x = 2, y = 8, R¹ = Me, R² = H).

2. 2-Alcényloxycarboxylates d'alcényle insaturés aux deux extrémités, de formule 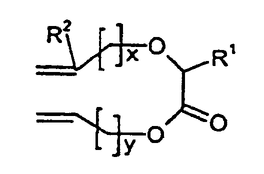 pour la préparation d'un composé selon la revendication 1,
dans lesquels
les composés ayant un centre chiral peuvent se trouver aussi bien sous forme (R) ou (S) que sous forme d'un mélange d'énantiomères,
R¹ et R² sont identiques ou différents et représentent un atome d'hydrogène ou un groupe alkyle inférieur,
x représente une chaîne alkylène saturée de 1 à 4 atomes de carbone et
y représente une chaîne alkylène saturée de 4 à 10 atomes de carbone,
le 2-alcényloxycarboxylate d'alcényle étant choisi dans le groupe constitué par les composés dans lesquels:
x = 1, y = 8, R¹ Me, R² = H,
x = 1, y = 8, R¹, R² = H,
x = 1, y = 9, R¹, R² = H,
x = 1, y = 9, R¹, R² = H,
x = 2, y = 8, R¹, R² = H
ou
x = 2, y = 8, R¹ = Me, R² = H.

3. Procédé de préparation de 1,4-dioxacycloalcan-2-ones ou de 1,4-dioxacycloalcén-2-ones macrocycliques selon la revendication 1, les composés ayant un centre chiral se trouvant sous forme de mélanges d'énantiomères,
**caractérisé en ce que** l'on utilise des acides alkylcarboxyliques pouvant être transformés en position 2 ou leurs esters de formule 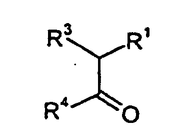 dans laquelle
R¹ a la signification indiquée dans la revendication 1 pour la 1,4-dioxacycloalcan-2-one ou la 1,4-dioxacycloalcén-2-one macrocyclique correspondante, et
R³ représente OH, Cl ou Br et
R⁴ représente OH, OMe ou OEt,
que l'on éthérifie dans une 1ère étape, estérifie dans une 2ème étape et cyclise par méthathèse d'oléfine dans une 3ème étape pour obtenir les 1,4-dioxacycloalcén-2-ones insaturées, que l'on hydrogène ensuite éventuellement dans une 4ème étape pour obtenir les 1,4-dioxacycloalcan-2-ones saturées.

4. Procédé de préparation de 3-alkyl-1,4-dioxacycloalcan-2-ones et de 3-alkyl-1,4-dioxacycloalcén-2-ones macrocycliques de formule 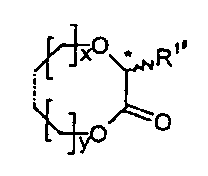 dans laquelle
la liaison en pointillés représente une liaison simple ou une double liaison E/Z, les composés pouvant se trouver sous forme E et Z dans le cas où il y a une double liaison dans le cycle, et les composés contenant un centre chiral se trouvant sous forme (R) ou (S),
R^{1'} a la signification indiquée dans la revendication 1 pour le reste R¹ d'une 1,4-dioxacycloalcan-2-one ou d'une 1,4-dioxacycloalcén-2-one macrocyclique correspondante, et
x a la signification indiquée dans la revendication 1 pour la 1,4-dioxacycloalcan-2-one ou la 1,4-dioxacycloalcén-2-one macrocyclique correspondante,
y a la signification indiquée dans la revendication 1 pour la 1,4-dioxacycloalcan-2-one ou la 1,4-dioxacycloalcén-2-one macrocyclique correspondante,
**caractérisé en ce que** l'on utilise comme produits de départ des esters d'acides (S)-2- ou (R)-2-hydroxycarboxyliques de formule 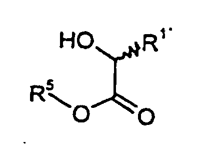 dans laquelle
R^{1'} a la signification indiquée ci-dessus et
R⁵ représente un reste alkyle en C₁-C₈,
que l'on éthérifie dans une 1ère étape dans des conditions acides non racémisantes, transestérifie dans une 2ème étape dans des conditions de catalyse avec un acide de Lewis et cyclise par méthathèse d'oléfine dans une 3ème étape pour obtenir les 3-alkyl-1,4-dioxacycloalcén-2-ones insaturées, que l'on hydrogène ensuite éventuellement dans une 4ème étape pour obtenir les 3-alkyl-1,4-dioxacycloalcan-2-ones saturées.

5. Parfums contenant des 1,4-dioxacycloalcan-2-ones et des 1,4-dioxacycloalcén-2-ones selon la revendication 1.

6. Compositions de parfums contenant des 1,4-dioxacycloalcan-2-ones et des 1,4-dioxacycloalcén-2-ones selon la revendication 1.

7. Compositions de parfums selon la revendication 6, contenant 1 à 40 % en masse de 1,4-dioxacycloalcan-2-ones et de 1,4-dioxacycloalcén-2-ones macrocycliques selon la revendication 1.

8. Utilisation de parfums selon la revendication 5 dans des compositions de parfums à caractère musqué.
